# EUROPEAN PATENT SPECIFICATION

(11) **EP 4 044 947 B1**
(45) Date of publication and mention of the grant of the patent: **12.06.2024**
(21) Application number: 20828482.8
(22) Date of filing: 03.12.2020
(51) Int. Cl.: A61B 18/14, A61N 1/32

(54) **ELECTROPORATION SYSTEM**
ELEKTROPORATIONSSYSTEM
SYSTÈME D'ÉLECTROPORATION

(30) Priority: 03.12.2019 US 201962943000 P
(43) Date of publication of application: 24.08.2022
(73) Proprietor: St. Jude Medical, Cardiology Division, Inc., St. Paul MN 55117 (US)
(72) Inventor: SUTERMEISTER, Derek, Ham Lake, MN 55304 (US); DALY, Jake, Ham Lake, MN 55304 (US); MOON, Boyce, Ham Lake, MN 55304 (US); BYRD, Israel, Richfield, Minnesota 55423 (US); OLSON, Eric, Maplewood, Minnesota 55119 (US); DREW, Kenneth, Brooklyn Park, Minnesota 55444 (US); TRANTER, John, Minneapolis, Minnesota 55417 (US); TEGG, Troy, Elk River, Minnesota 55330 (US)
(74) Representative: Alpspitz IP
(86) International application number: PCT/US2020/063039
(87) International publication number: WO 2021/113463

(56) References cited:
- WO-A1-2018/208795
- WO-A1-2019/133608
- WO-A1-2020/061192
- WO-A1-2021/127558
- WO-A1-2021/173215
- WO-A1-2021/247738
- US-A1- 2008 027 428
- US-A1- 2010 261 994
- US-A1- 2016 367 310
- US-A1- 2018 071 014
- US-A1- 2018 214 202
- US-A1- 2019 143 106

## Description

### BACKGROUND OF THE DISCLOSURE

### a. Field of the Disclosure

The present disclosure relates generally to medical devices that are used in the human body. In particular, the present disclosure relates to electroporation systems and methods of controlling electroporation systems. The invention is set out in the appended claims.

### b. Background

Various therapies are used to treat various conditions afflicting the human anatomy. Cardiac arrhythmias, for example are sometimes treated using ablation therapy. When tissue is ablated, or at least subjected to ablative energy generated by an ablation generator and delivered by an ablation catheter, lesions form in the tissue. Electrodes mounted on or in ablation catheters are used to create tissue necrosis in cardiac tissue to correct conditions such as atrial arrhythmia (including, but not limited to, ectopic atrial tachycardia, atrial fibrillation, and atrial flutter). Arrhythmia (i.e., irregular heart rhythm) can create a variety of dangerous conditions including loss of synchronous atrioventricular contractions and stasis of blood flow that can lead to a variety of ailments and even death. It is believed that the primary cause of atrial arrhythmia is stray electrical signals within the left or right atrium of the heart. The ablation catheter imparts ablative energy (e.g., radiofrequency energy, cryoablation, lasers, chemicals, high-intensity focused ultrasound, etc.) to cardiac tissue to create a lesion in the cardiac tissue. This lesion disrupts undesirable electrical pathways and thereby limits or prevents stray electrical signals that lead to arrhythmias.

One candidate for use in therapy of cardiac arrhythmias is electroporation. Electroporation therapy involves electric field-induced pore formation on the cell membrane. The electric field may be induced by applying a direct current (DC) signal delivered as a relatively short-duration pulse. Such a pulse may be repeated to form a pulse train. When such an electric field is applied to tissue *in vivo,* the cells in the tissue are subjected to trans-membrane potential, which opens the pores on the cell wall, hence the term electroporation. Electroporation may be reversible (i.e., the temporally-opened pores will reseal) or irreversible (i.e., the pores will remain open). For example, in the field of gene therapy, reversible electroporation (i.e., temporarily open pores) is used to transfect high molecular weight therapeutic vectors into the cells. In other therapeutic applications, a suitably configured pulse train alone may be used to cause cell destruction, for instance by causing irreversible electroporation (IRE).

US 2018/071014 A1 relates to a treatment device and method for delivering electrical pulses capable of creating irreversible electroporation by using pulse width range of 50 microseconds to 100 microseconds.

US 2010/261994 A1 relates to methods for destroying aberrant cells using a series of electrical pulses having durations on the nanosecond scale.

US 2019/143106 A1 relates to irreversible electroporation through a combination of substance injection and electrical field application without using an electrode loop assembly or a planar electrode assembly.

WO 2019/133608 A1 relates to devices for transmitting energy to a body lumen or passageway in the form of pulsed electric fields, wherein no electrode loop assembly or a planar electrode assembly is used.

### SUMMARY OF THE DISCLOSURE

The present disclosure is directed to an electroporation system including a catheter shaft, at least one electrode coupled to the catheter shaft at a distal end thereof, and an electroporation generator coupled in communication with the at least one electrode. The electroporation generator configured to supply a biphasic pulse signal to the at least one electrode. The biphasic pulse signal includes a first phase having a first polarity and a first pulse duration, and a second phase having a second polarity opposite to the first polarity, and a second pulse duration. Each of the first phase and second phase has a voltage amplitude of at least 500 volts and a pulse duration of less than 20 microseconds. The second phase is generated at a non-zero interval following the first phase.

The present disclosure is further directed to a non-claimed method including supplying, by an electroporation generator, a first phase of a biphasic pulse signal to at least one electrode coupled at a distal end of a catheter shaft. The first phase has a first polarity and a first pulse duration. The method further includes supplying a second phase of the biphasic pulse signal to the at least one electrode. The second phase has a second polarity opposite to the first polarity, and a second pulse duration. Each of the first phase and second phase has a voltage amplitude of at least 500 volts and a pulse duration of less than 20 microseconds. The second phase is generated at a non-zero interval following the first phase.

The present disclosure is further directed to an electroporation generator including a positive high-voltage direct current (+HVDC) supply having a first polarity, a negative high-voltage direct current (-HVDC) supply having a second polarity opposite the first polarity, a plurality of semiconductor switches connected in a bridge configuration to regulate application of the +HVDC supply and the -HVDC supply to first and second conductors for a catheter, and a microcontroller communicatively coupled to the plurality of semiconductor switches. The microcontroller is configured to control commutation of the plurality of semiconductor switches to transmit a biphasic pulse signal through the first and second conductors for the catheter.

The present disclosure is further directed to a method of generating a pulse signal. The method includes supplying a positive high-voltage direct current (+HVDC) supply having a first polarity to a plurality of semiconductor switches connected in a bridge configuration, supplying a negative high-voltage direct current (-HVDC) supply having a second polarity opposite the first polarity to the plurality of semiconductors, and commutating the plurality of semiconductor switches to apply the +HVDC supply to a first conductor of a catheter and to apply the -HVDC supply to a second conductor of the catheter for a first duration. The method further includes commutating the plurality of semiconductor switches, after the first duration, to electrically disconnect the first conductor and the second conductor from the +HVDC supply and the -HVDC supply for a second duration, and commutating the plurality of semiconductor switches, after the second duration, to apply the +HVDC supply to the second conductor and to apply the -HVDC supply to the first conductor for a third duration. The method further includes commutating the plurality of semiconductor switches, after the third duration, to electrically disconnect the first conductor and the second conductor from the +HVDC supply and the -HVDC supply. The present invention is set out in the appended claims.

### BRIEF DESCRIPTION OF THE DRAWINGS

FIG. 1 is a schematic and block diagram view of a system for electroporation therapy.
FIG. 2 is a side view of an exemplary electrode assembly suitable for use in the system of FIG. 1, illustrated in the form of an electrode loop assembly.
FIG. 3 is an end view of the electrode loop assembly of FIG. 2.
FIG. 4 is a perspective view of another exemplary electrode assembly suitable for use in the system of FIG. 1, illustrated in the form of a basket electrode assembly.
FIG. 5 is a perspective view of another exemplary electrode assembly suitable for use in the system of FIG. 1, illustrated in the form of a grid electrode assembly.
FIG. 6 is a side view of another exemplary electrode assembly suitable for use in the system of FIG. 1, illustrated in the form of an expandable electrode assembly.
FIG. 7 is a plot illustrating an exemplary pulse signal that may be generated by an electroporation generator of the system of FIG. 1.
FIG. 8 is plot illustrating a burst signal that includes two pulse signals generated at a pulse period.
FIG. 9 is plot illustrating multiple burst signals generated at a repeating burst period.
FIG. 10 is a schematic diagram of an exemplary electroporation generator for use in the system of FIG. 1.
FIG. 11 is a flow diagram of an exemplary method of generating a pulse signal, such as the pulse signal shown in FIG. 7.

Corresponding reference characters indicate corresponding parts throughout the several views of the drawings. It is understood that that Figures are not necessarily to scale.

### DETAILED DESCRIPTION OF THE DISCLOSURE

The present disclosure relates generally to medical devices that are used in the human body. In particular, in many embodiments, the present disclosure relates to electroporation systems and methods for controlling such electroporation systems. The disclosed embodiments may lead to more consistent and improved patient outcomes in electroporation therapy procedures. For example, embodiments of the present disclosure utilize electroporation pulse signals having specific parameters (e.g., voltage amplitude, pulse width or duration, pulse period, and burst period) that facilitate reducing or minimizing undesirable or unintended IRE, such as skeletal muscle excitation and generation of gasses within a patient. It is contemplated, however, that the described features and methods of the present disclosure as described herein may be incorporated into any number of systems as would be appreciated by one of ordinary skill in the art based on the disclosure herein.

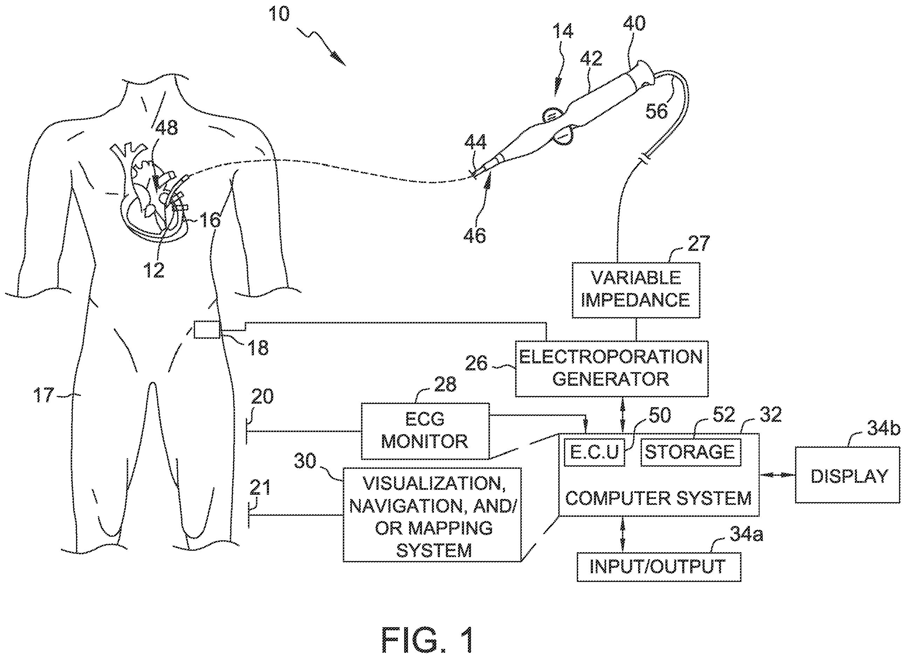

Referring now to the drawings, FIG. 1 illustrates an exemplary embodiment of a system 10 for electroporation therapy. In general, the various embodiments include an electrode assembly disposed at the distal end of a catheter. As used herein, "proximal" refers to a direction toward the end of the catheter near the clinician and "distal" refers to a direction away from the clinician and (generally) inside the body of a patient. The electrode assembly includes one or more individual, electrically-isolated electrode elements. In some embodiments, each electrode element, also referred to herein as a catheter electrode, is individually wired such that it can be selectively paired or combined with any other electrode element to act as a bipolar or a multi-polar electrode.

System 10 may be used for irreversible electroporation (IRE) to destroy tissue. In particular, system 10 may be used for electroporation-induced primary necrosis therapy, which refers to the effects of delivering electrical current in such manner as to directly cause an irreversible loss of plasma membrane (cell wall) integrity leading to its breakdown and cell necrosis. This mechanism of cell death may be viewed as an "outside-in" process, meaning that the disruption of the outside wall of the cell causes detrimental effects to the inside of the cell. Typically, for typical plasma membrane electroporation, electric current is delivered as a pulsed electric field in the form of short-duration direct current (DC) pulses between closely spaced electrodes capable of delivering an electric field strength sufficient to cause irreversible electroporation in the targeted cells. As described in more detail herein, the system 10 is configured to deliver an electroporation pulse signal having a relatively high voltage and low pulse duration as compared to at least some prior electroporation systems. The waveforms generated by system 10 and applied to catheter electrodes facilitate reducing and/or preventing skeletal muscle stimulation during IRE therapy.

Irreversible electroporation through a multi electrode hoop catheter may enable pulmonary vein isolation in as few as one shock per vein, which may produce much shorter procedure times compared to sequentially positioning a radiofrequency (RF) ablation tip around a vein. It should be understood that the mechanism of cell destruction in electroporation is not primarily due to heating effects, but rather to cell membrane disruption through application of a high-voltage electric field. Thus, electroporation may avoid some possible thermal effects that may occur when using RF energy. This "cold" or "non-thermal" therapy thus has desirable characteristics.

It should be understood that while the energization strategies are described as involving DC pulses, embodiments may use variations of DC pulses and remain within the spirit and scope of the invention. For example, exponentially-decaying pulses, exponentially-increasing pulses, and combinations thereof may be used. Moreover, while system 10 is described herein with respect to IRE ablation therapy, it should be understood that system 10 may be used, additionally or alternatively, for other forms of ablation therapy, including, for example and without limitation, radiofrequency (RF) ablation.

System 10 includes a catheter electrode assembly 12 including at least one catheter electrode configured to be used as briefly outlined above and as described in greater detail below. Electrode assembly 12 is incorporated as part of a medical device such as a catheter 14 for electroporation therapy of tissue 16 in a body 17 of a patient. In the illustrative embodiment, tissue 16 comprises heart or cardiac tissue. It should be understood, however, that embodiments may be used to conduct electroporation therapy with respect to a variety of other body tissues.

FIG. 1 further shows a plurality of return electrodes designated 18, 20, and 21 that are diagrammatic of the body connections that may be used by the various sub-systems included in the overall system 10, such as an electroporation generator 26, an electrophysiology (EP) monitor such as an ECG monitor 28, a visualization, navigation, and/or mapping system 30 for visualization, mapping and navigation of internal body structures. In the illustrated embodiment, return electrodes 18, 20, and 21 are patch electrodes. It should be understood that the illustration of a single patch electrode is diagrammatic only (for clarity), and that such sub-systems to which these patch electrodes are connected may, and typically will, include more than one patch (body surface) electrode. In other embodiments, return electrodes 18, 20, and 21 may be any other type of electrode suitable for use as a return electrode including, for example, one or more catheter electrodes. Return electrodes that are catheter electrodes may be part of electrode assembly 12 or part of a separate catheter (not shown). In some embodiments, for example, system 10 includes a bipolar catheter electrode assembly that includes a plurality of electrode pairs, where each electrode pair includes two electrodes with one electrode functioning as the return electrode.

System 10 may further include a main computer system 32 (including an electronic control unit 50 and data storage-memory 52), which may be integrated with system 30 in certain embodiments. System 32 may further include conventional interface components, such as various user input/output mechanisms 34a and a display 34b, among other components.

In some embodiments, electroporation generator 26 and/or computer system 32 may be programmed or otherwise configured to run an algorithm that identifies and/or selects which electrodes or electrode pairs of electrode assembly 12 to energize. That is, electrodes or electrode pairs of electrode assembly 12 may be selectively energized based on, for example, anatomical location of the electrode(s) and/or contact between the electrode(s) and tissue 16. For example, system 10 may include a suitable detector and tissue sensing circuit that identify which electrodes of electrode assembly 12 have characteristics (e.g., if electrical characteristics, then for example, impedance, phase angle, reactance, etc.) indicative of contact with tissue 16. Electroporation generator 26 and/or computer system 32 may then select which electrodes or electrode pairs of catheter assembly 12 to energize based on the electrodes identified as being in contact with tissue 16. By way of example, if a basket catheter is inserted into the antrum of a pulmonary vein, electroporation generator 26 and/or computer system 32 may determine which electrode(s) to activate based on contact with tissue 16, or even the specific anatomical location within the heart. Suitable components and methods for identifying electrodes in contact with tissue are described, for example, in U.S. Patent No. 9,289,606, the disclosure of which is incorporated herein by reference in its entirety.

In the illustrative embodiment, catheter 14 includes a cable connector 40, or interface, a handle 42, and a shaft 44 having a proximal end 46 and a distal end 48. Catheter 14 may also include other conventional components not illustrated herein such as a temperature sensor, additional electrodes, and corresponding conductors or leads. The connector 40 provides mechanical and electrical connection(s) for cable 56 extending from generator 26. The connector 40 may comprise conventional components known in the art and, as shown, is disposed at the proximal end of catheter 14.

Handle 42 provides a location for the clinician to hold catheter 14 and may further provide means for steering or guiding shaft 44 within body 17. For example, handle 42 may include means to change the length of a guidewire extending through catheter 14 to distal end 48 of shaft 44 or means to steer shaft 44. Moreover, in some embodiments, handle 42 may be configured to vary the shape, size, and/or orientation of a portion of the catheter. Handle 42 is also conventional in the art and it will be understood that the construction of handle 42 may vary. In an alternate exemplary embodiment, catheter 14 may be robotically driven or controlled. Accordingly, rather than a clinician manipulating a handle to advance/retract and/or steer or guide catheter 14 (and shaft 44 thereof in particular), a robot is used to manipulate catheter 14. Shaft 44 is an elongated, tubular, flexible member configured for movement within body 17. Shaft 44 is configured to support electrode assembly 12 as well as contain associated conductors, and possibly additional electronics used for signal processing or conditioning. Shaft 44 may also permit transport, delivery and/or removal of fluids (including irrigation fluids and bodily fluids), medicines, and/or surgical tools or instruments. Shaft 44 may be made from conventional materials such as polyurethane and defines one or more lumens configured to house and/or transport electrical conductors, fluids or surgical tools. Shaft 44 may be introduced into a blood vessel or other structure within body 17 through a conventional introducer. Shaft 44 may then be advanced, retracted and/or steered or guided through body 17 to a desired location such as the site of tissue 16, including through the use of guidewires or other means known in the art.

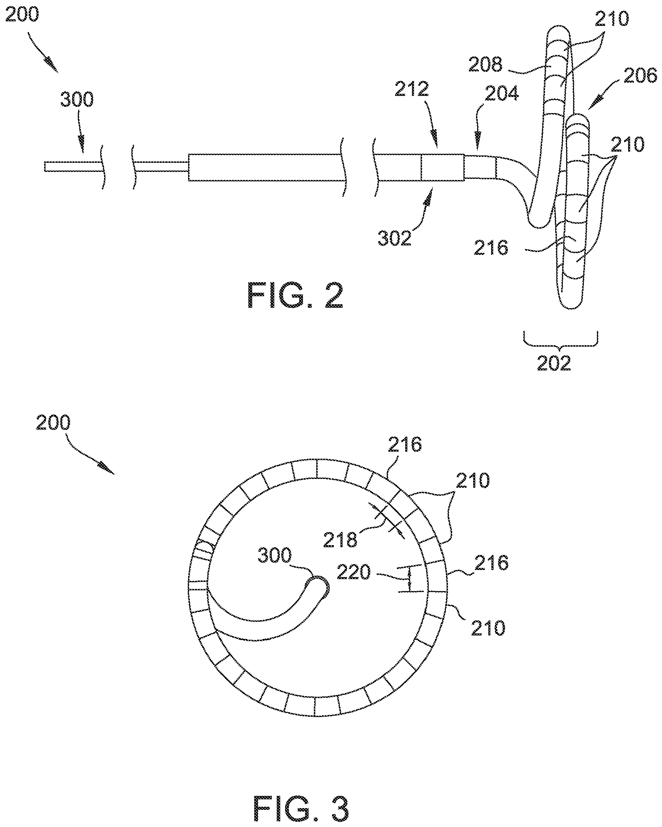

In some embodiments, catheter 14 is a hoop catheter (shown, for example, in FIGS. 2 and 3), sometimes referred to as a spiral or loop catheter, having catheter electrodes distributed about one or more hoops at the distal end of shaft 44. The diameter of the hoop(s) (sometimes referred to herein as "loops") may be variable. In some embodiments, the hoop catheter diameter is variable by about ten millimeters (mm) between a minimum diameter and a maximum diameter. The minimum diameter in some embodiments may be selected between about thirteen mm and about twenty mm when the catheter 14 is manufactured. With a ten mm range of variability, such catheters would have a maximum diameter between twenty-three mm and thirty mm. In other embodiments, the hoop diameter is variable between about fifteen mm and about twenty eight mm, between about thirteen mm and about twenty-three mm, or between about seventeen mm and about twenty-seven mm. Alternatively, the catheter may be a fixed diameter hoop catheter or may be variable between different diameters. In some embodiments, catheter 14 has fourteen catheter electrodes (e.g., grouped as seven pairs of catheter electrodes). In other embodiments, catheter 14 includes ten catheter electrodes, twenty catheter electrodes, or any other suitable number of electrodes for performing electroporation. In some embodiments, the catheter electrodes are ring electrodes. Alternatively, the catheter electrodes may be any other suitable type of electrodes, such as single sided electrodes or electrodes printed on a flex material. In various embodiments, the catheter electrodes have lengths of 1.0 mm, 2.0 mm, 2.5 mm, and/or any other suitable length for electroporation.

FIGS. 2 and 3 illustrate an exemplary electrode assembly 12 suitable for use in system 10, illustrated in the form of an electrode hoop or loop assembly 200. FIG. 2 is a side view of electrode loop assembly 200 with a variable diameter loop 202 coupled at the distal end 302 of a catheter shaft 300. FIG. 3 is an end view of variable diameter loop 202 of electrode loop assembly 200. As shown in FIGS. 2 and 3, electrode loop assembly 200 extends from a proximal end 204 to a distal end 206, and includes an outer sleeve 208 formed in the shape of a loop, and a plurality of catheter electrodes 210 mounted on outer sleeve 208. Proximal end 204 of electrode loop assembly 200 is coupled to catheter shaft 300 via a suitable coupler 212. Electrodes 210 may be used for a variety of diagnostic and therapeutic purposes including, for example and without limitation, cardiac mapping and/or ablation (e.g., IRE ablation). For example, electrode loop assembly 200 may be configured as a bipolar electrode assembly for use in bipolar-based electroporation therapy. More specifically, electrodes 210 may be configured as electrode pairs (e.g., cathode-anode electrode pairs) and electrically coupled to electroporation generator 26 (e.g., via suitable electrical wire or other suitable electrical conductors extending through catheter shaft 44) such that adjacent electrodes 210 are energized with opposite polarities to generate a potential and corresponding electric field between adjacent electrodes 210. In other embodiments, any combination of electrodes 210 may be configured as electrode pairs (e.g., cathode-anode electrode pairs), including, for example and without limitation, adjacent electrodes, non-adjacent electrodes, and any other combination of electrodes that enables system 10 to function as described herein. As described above, for example, electroporation generator 26 and/or computer system 32 may selectively energize certain electrodes 210 of electrode loop assembly 200 to form electrode pairs based on contact between electrodes 210 and tissue 16. In yet other embodiments, electrode loop assembly 200 may be configured other than as a bipolar electrode assembly, such as a unipolar or monopolar electrode assembly. In such embodiments, electrode 18 may function as the return electrode.

In the illustrated embodiment, variable diameter loop 202 includes fourteen catheter electrodes 210 evenly spaced around the circumference of variable diameter loop 202. In other embodiments, variable diameter loop 202 may include any suitable number of catheter electrodes 210 made of any suitable material. Each catheter electrode 210 is separated from each other catheter electrode by an insulated gap 216. In the example embodiment, each catheter electrode 210 has a same length 218 (shown in FIG. 3) and each insulated gap 216 has a same length 220 as each other gap 216. Length 218 and length 220 are both about 2.5 mm in the example embodiment. In other embodiments, length 218 and length 220 may be different from each other. Moreover, in some embodiments, catheter electrodes 210 may not all have the same length 218 and/or insulated gaps 216 may not all have the same length 220. In some embodiments, catheter electrodes 210 are not spaced evenly around the circumference of variable diameter loop 202.

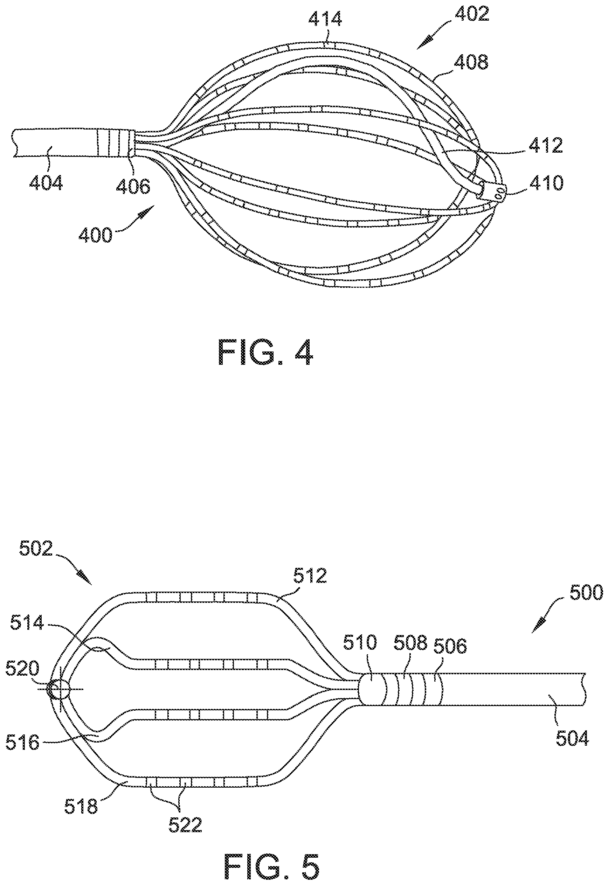

FIG. 4 is a perspective view of another exemplary electrode assembly 12 suitable for use in system 10, illustrated in the form of a basket electrode assembly 400. Basket electrode assembly 400 includes a basket 402 coupled to a catheter body 404 by a suitable proximal connector 406. Basket 402 includes a plurality of splines 408 and a distal coupler 410 at which each of splines 408 terminates. In some embodiments, such as the illustrated embodiment, basket electrode assembly 400 may also include irrigation tubing 412 (e.g., to supply fluids to basket electrode assembly 400). In other embodiments, irrigation tubing 412 may be omitted. Each of the plurality of splines 408 includes at least one electrode 414. In the illustrated embodiment, each of the plurality of splines includes eight electrodes 414, although each spline 408 may include more than or less than eight electrodes 414.

Electrodes 414 may be used for a variety of diagnostic and therapeutic purposes including, for example and without limitation, cardiac mapping and/or ablation (e.g., IRE ablation). For example, basket electrode assembly 400 may be configured as a bipolar electrode assembly for use in bipolar-based electroporation based electroporation. More specifically, electrodes 414 positioned on adjacent splines 408 may be configured as electrode pairs (e.g., cathode-anode electrode pairs) and electrically coupled to electroporation generator 26 (e.g., via suitable electrical wire or other suitable electrical conductors extending through catheter shaft 44) such that electrodes 414 on adjacent splines 408 are energized with opposite polarities to generate a potential and corresponding electric field between electrodes 414 of adjacent splines 408. In other embodiments, electrodes 414 positioned along the same spline 408 may be configured as electrode pairs. In yet other embodiments, any combination of electrodes 414 may be configured as electrode pairs, including, for example and without limitation, adjacent electrodes, non-adjacent electrodes, electrodes on adjacent splines, electrodes on non-adjacent splines, and any other combination of electrodes that enables system 10 to function as described herein. As described above, for example, electroporation generator 26 and/or computer system 32 may selectively energize certain electrodes 414 of basket electrode assembly 400 to form electrode pairs based on contact between electrodes 414 and tissue 16. In other embodiments, basket electrode assembly 400 may be configured other than as a bipolar electrode assembly, such as a unipolar or monopolar electrode assembly. In such embodiments, electrode 18 may function as the return electrode.

FIG. 5 is a perspective view of another exemplary electrode assembly 12 suitable for use in system 10, illustrated in the form of a planar or grid electrode assembly 500. Grid electrode assembly 500 includes a paddle 502 coupled to a catheter body 504. In the illustrated embodiment, catheter body 504 includes body electrodes 506, 508, and 510 coupled thereto. In the illustrated embodiment, paddle 502 includes a first spline 512, a second spline 514, a third spline 516, and a fourth spline 518 coupled to catheter body 504 by a proximal coupler and coupled to each other by a distal connector 520 at a distal end of paddle 502. In one embodiment, first spline 512 and fourth spline 518 can be one continuous segment, and second spline 514 and third spline 516 can be another continuous segment. In other embodiments the various splines can be separate segments coupled to each other. The plurality of splines can further comprise a varying number of electrodes 522. The electrodes in the illustrated embodiment can comprise ring electrodes evenly spaced along the splines. In other embodiments the electrodes can be evenly or unevenly spaced and the electrodes can comprise point or other types of electrodes.

Electrodes 522 may be used for a variety of diagnostic and therapeutic purposes including, for example and without limitation, cardiac mapping and/or ablation (e.g., IRE ablation). For example, grid electrode assembly 500 may be configured as a bipolar electrode assembly for use in bipolar-based electroporation based electroporation. More specifically, adjacent electrodes 522 may be configured as electrode pairs (e.g., cathode-anode electrode pairs) and electrically coupled to electroporation generator 26 (e.g., via suitable electrical wire or other suitable electrical conductors extending through catheter shaft 44) such that adjacent electrodes 522 are energized with opposite polarities to generate a potential and corresponding electric field between adjacent electrodes 522. Adjacent electrodes 522 that form a bipole pair may be located along the same spline (e.g., electrodes 522 along first spine 512) or across adjacent splines. In one embodiment, for example, electrode pairs may be formed between one electrode 522 (e.g., a cathode) located on first spline 512 and another, adjacent electrode 522 (e.g., an anode) located on adjacent second spline 514. In other embodiments, any combination of electrodes 522 may be configured as electrode pairs (e.g., cathode-anode electrode pairs), including, for example and without limitation, adjacent electrodes, non-adjacent electrodes, electrodes on adjacent splines, electrodes on non-adjacent splines, and any other combination of electrodes that enables system 10 to function as described herein. As described above, for example, electroporation generator 26 and/or computer system 32 may selectively energize certain electrodes 522 of grid electrode assembly 500 to form electrode pairs based on contact between electrodes 522 and tissue 16. In other embodiments, grid electrode assembly 500 may be configured other than as a bipolar electrode assembly, such as a unipolar or monopolar electrode assembly. In such embodiments, electrode 18 may function as the return electrode.

First spline 512, second spline 514, third spline 516, and fourth spline 518 are generally aligned in the same (topological) plane. Although paddle 502 is illustrated as relatively flat or planar in FIG. 5, it should be understood that paddle 502 may bend, curl, buckle, twist, and/or otherwise deform. Accordingly, the plane defined by paddle 502 and splines 512, 514, 516, and 518 may correspondingly deform, such that the plane is a non-flat topological plane.

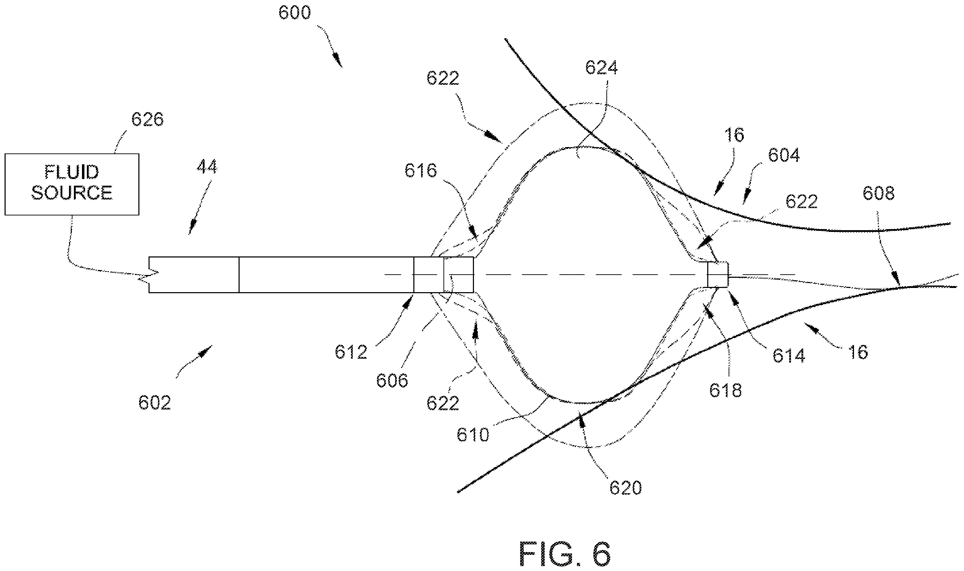

FIG. 6 is a perspective view of another exemplary electrode assembly 12 suitable for use in system 10, illustrated in the form of an expandable electrode assembly 600. Electrode assembly 600 extends axially from a proximal end 602 of electrode assembly 600 to a distal end 604 of electrode assembly 600, generally along a longitudinal axis 606. Proximal end 602 is coupled to catheter shaft 44 (e.g., to a distal end of shaft 44) via a suitable coupler (not shown). In the exemplary embodiment, a guidewire 608 extends axially through shaft 44 and through electrode assembly 600. Guidewire 608 may be manipulated (e.g., using handle 42) to adjust a position of electrode assembly 600 within body 17.

Electrode assembly 600 generally includes an expandable isolation member 610 and a pair of electrodes 612, 614. More specifically, expandable isolation member 610 extends between a proximal end 616 of expandable isolation member 610 and a distal end 618 of expandable isolation member 610. Electrodes 612, 614 are arranged adjacent proximal end 616 and distal end 618 of expandable isolation member 610, respectively, such that expandable isolation member 610 is disposed axially between electrodes 612, 614. A proximal electrode 612 is adjacent proximal end 616 of expandable isolation member 610 and is proximate to proximal end 602 of electrode assembly 600. Likewise, a distal electrode 614 is adjacent distal end 618 of expandable isolation member 610 and is proximate to distal end 604 of electrode assembly 600. Distal end 618 of expandable isolation member 610 is proximal to distal end 604 of electrode assembly 600 in the exemplary embodiment.

Electrodes 612, 614 may be used for a variety of diagnostic and therapeutic purposes including, for example and without limitation, cardiac mapping and/or ablation (e.g., IRE ablation). For example, electrode assembly 600 may be configured as a bipolar electrode assembly for use in bipolar-based electroporation therapy. Specifically, electrodes 612, 614 can be individually electrically coupled to generator 26 (e.g., via suitable electrical wire or other suitable electrical conductors extending through catheter shaft 44) and configured to be selectively energized (e.g., by electroporation generator 26 and/or computer system 32) with opposite polarities to generate a potential and corresponding electric field therebetween, for IRE therapy. That is, one of electrodes 612, 614 is configured to function as a cathode, and the other is configured to function as an anode. Electrodes 612, 614 may be any suitable electroporation electrodes. In the exemplary embodiment, electrodes 612, 614 are ring electrodes. Electrodes 612, 614 may have any other shape or configuration. It is realized that the shape, size, and/or configuration of electrodes 612, 614 may impact various parameters of the applied electroporation therapy. For example, increasing the surface area of one or both electrodes 612, 614 may reduce the applied voltage needed to cause the same level of tissue destruction. Moreover, although each of proximal electrode 612 and distal electrode 614 are illustrated as single electrodes, either or both of proximal electrode 612 and distal electrode 614 may be alternatively embodied as two or more discrete electrodes. Further, while electrode assembly 600 is described as a bipolar electrode assembly, it should be understood that in some embodiments, electrode assembly 600 may configured as a unipolar or monopolar electrode assembly and use a patch electrode (e.g., return electrode 18) as a return or indifferent electrode.

In the exemplary embodiment, expandable isolation member 610 is configurable between a collapsed configuration (not shown) and an expanded configuration (as shown in FIG. 6). For example, expandable isolation member 610 is delivered in the collapsed configuration to the target location of tissue 16 within body 17 (e.g., axially disposed within catheter shaft 44) and transitioned to the expanded configuration at the target location. Expandable isolation member 610 is configured to sealingly engage with tissue 16 at the target location and to inhibit electrical communication between electrodes 612, 614 (e.g., by at least partially insulating distal electrode 614 from proximal electrode 612). More specifically, expandable isolation member 610, is formed from an electrically insulating material. Therefore, a current flow 622 between proximal electrode 612 and distal electrode 614 is diverted around expandable isolation member 610. FIG. 6 depicts a plurality of current flows 622 of varying shape and magnitude of diversion around expandable isolation member 610. In some embodiments, the shape and size of expandable isolation member 610 may be selected to influence current flow 622 therearound (e.g., a magnitude of diversion, a shape or direction of the resulting current flow 622, etc.).

Moreover, expandable isolation member 610 is configured to sealingly engage tissue 16 when in the expanded configuration. In one exemplary embodiment, expandable isolation member 610 includes a circumferential sealing surface 620 configured for sealing engagement with tissue 16 such that expandable isolation member 610 inhibits fluid communication and, consequently, electrical communication (e.g., current flow), between the electrodes 612, 614 when engaged with tissue of the patient. For example, where expandable isolation member 610 is used for pulmonary vein isolation (PVI) or to isolate other cylindrical or tubular tissue (e.g., other vasculature tissue), expandable isolation member 610 inhibits or substantially prevents the flow of blood therearound. Therefore, when electrodes 612, 614 are energized, current 622 flows therebetween through tissue 16 adjacent expandable isolation member 610, rather than through blood. In this way, the electroporation therapy may be more localized and, therefore, require reduced applied voltage to cause the desired amount of cell destruction. Specifically, fluid (e.g., blood) is more electrically conductive than tissue, therefore current flows through blood more readily than through tissue, and electroporation therapy is less effective. By blocking the blood flow, the current 622 between electrodes 612, 614 is diverted through adjacent tissue 16, thereby increasing the effectiveness of electroporation therapy at a given voltage.

In the exemplary embodiment, expandable isolation member 610 includes an outer layer 624 formed or constructed from an electrically insulating material. For example, outer layer 624 may include polyethylene terephthalate (PET). Outer layer 624 made include any other suitable material that is electrically insulating and able to accommodate expansion and contraction of electrode assembly 600. In certain embodiments, as shown in FIG. 6, expandable isolation member 610 is embodied as an inflatable balloon. In such embodiments, the inflatable balloon is coupled to a fluid source 626 for selectively inflating the balloon (e.g., when electrode assembly 600 has been advanced to the target location within body 17 and has been deployed from catheter shaft 44). In some embodiments, the fluid source includes a dielectric fluid, such as deionized water, saline, carbon dioxide gas, nitrous oxide gas, and/or air. In other embodiments, expandable isolation member 610 may be selectively expanded using other means, such as an expandable frame (e.g., a frame formed from a shape-memory material) retained within outer layer 624.

Although expandable isolation member 610 is shown in FIG. 6 as having an elongated spherical shape, expandable isolation member 610 may have any other shape or configuration the enables sealing - and, therefore, inhibiting fluid and/or electrical communication between electrodes 612, 614. The particular shape and/or configuration may be selected for the particular tissue isolation desired. For example, in other embodiments, isolation and tissue destruction within solid or planar tissue, such as the wall of a heart chamber (as opposed to the relatively cylindrical isolation of a vessel), may be desired. In such embodiments, distal end 618 of expandable isolation member 610 may be inverted and concave, and distal electrode 614 is positioned within the concavity of distal end 618. Distal end 618 may be engaged or pressed against the tissue to seal or isolate distal electrode 614 from proximal electrode 612, such that current flow 622 between electrodes 612, 614 is diverted through the tissue (e.g., of the heart chamber wall) engaged with distal end 618.

It is contemplated that full sealing between expandable isolation member 610 and the adjacent tissue 16 may not occur. For example, circumferential sealing surface 620 may not be fully engaged with tissue 16, and some fluid (blood) flow past circumferential sealing surface 620 may occur. In some embodiments, complete engagement or sealing is not necessary for electroporation therapy to proceed successfully. The level of sealing may be ascertained using a variety of methods. In some embodiments, introducing fluoroscopic contrast materials are introduced into the blood stream upstream of expandable isolation member 610, and the presence or amount of contrast material downstream of expandable isolation member 610 is determined using x-rays. In other embodiments, Doppler ultrasound is used to determine the level of fluid flow past expandable isolation member 610. In still other embodiments, impedance between electrodes 612, 614 is measured before and after placement of electrode assembly 600 at the target location; a threshold shift in impedance reflects sufficient sealing. In yet other embodiments, electrode assembly 600 includes a pressure transducer (not shown) on distal end 604 that is used to measure fluid pressure to reflect the level of sealing between expandable isolation member 610 and tissue 16. Additional and/or alternative methods to determine the level of sealing may be used. Moreover, any of the above-described methods can be employed iteratively. Specifically, an initial level of sealing may be determined, and, in response, a position of electrode assembly 600 may be adjusted. A subsequent level of sealing may be determined, and so forth, until an adequate or sufficient level of sealing is reached (e.g., based on threshold values and/or physician determination).

Moreover, based on the determined level of sealing using any of the above methods (or any other suitable method), an appropriate level of voltage to be applied may be selected. A reduced level of sealing may require an increased applied voltage.

It should be understood that electrode assembly 12 is not limited to the specific constructions shown and described herein, and may include any other suitable electrode assembly and have any other suitable construction that enables system 10 to function as described herein. By way of example, electrode assembly 12 may have the same or similar construction as electrode assemblies described in U.S. Patent No. 10,136,829, U.S. Patent Application Publication Nos. 2018/0014751 and 2019/0201688, International Patent Application Publication No. WO2018/208795, and U.S. Provisional Patent Application Serial Nos. 62/861,135, 62/842,654, and 62/983,200.

Referring again to FIG. 1, the visualization, navigation, and/or mapping system 30 can comprise an electric field-based system, or, sometimes referred to as an impedance based system, such as, for example, that having the model name EnSite NAVX^{™} system and commercially available from Abbott Laboratories, and as generally shown with reference to U.S. Pat. No. 7,263,397 titled "Method and Apparatus for Catheter Navigation and Location and Mapping in the Heart". The visualization, navigation, and/or mapping system 30 can also include other commercially available systems, including the EnSite^{™} Velocity^{™} or EnSite Precision^{™} cardiac mapping and visualization systems of Abbott Laboratories. In other exemplary embodiments, the visualization, navigation, and/or mapping system 30 can comprise other types of systems, such as, for example and without limitation: a magnetic field-based system such as the CARTO System (now in a hybrid form with impedance- and magnetically-driven electrodes) available from Biosense Webster, or the gMPS system from MediGuide Ltd. In accordance with a combination electric field-based and magnetic field-based system, the catheter can include both electrodes as impedance-based electrodes and one or more magnetic field sensing coils. Commonly available fluoroscopic, computed tomography (CT), and magnetic resonance imaging (MRI)-based systems can also be used.

Electroporation generator 26 is configured to energize the electrode element(s) in accordance with an electroporation energization strategy, which may be predetermined or may be user-selectable. For electroporation-induced primary necrosis therapy, generator 26 may be configured to produce an electric current that is delivered via electrode assembly 12 as a pulsed electric field in the form of short-duration DC pulses transmitted between closely spaced electrodes (e.g., electrode pairs of electrode assembly 12) and capable of delivering an electric field strength of about 0.1 to 1.0 kV/cm (e.g., at the tissue site). The voltage amplitude and pulse duration needed for irreversible electroporation are inversely related. For example, as pulse durations are decreased, the voltage amplitude must be increased to achieve electroporation.

In some embodiments, the electrodes of electrode assembly 12 may be energized sequentially such that only some of electrodes are energized at a given time. That is, not all electrodes of electrode assembly 12 are energized simultaneously. In some embodiments, for example, a first pair of electrodes may be energized according to an electroporation energization strategy, and subsequently, a second pair of electrodes may be energized according to the electroporation energization strategy. The sequential energization of electrodes may continue on to a third pair of electrodes, a fourth pair of electrodes, and so on. The pairs of electrodes may include adjacent or non-adjacent electrodes. By way of example, where an electrode assembly includes a plurality of electrodes sequentially numbered 1 through n according to position (i.e., the second electrode is adjacent to the first electrode and the third electrode), the electrodes may be sequentially energized as pairs by energizing, in sequence, the first and second electrodes, the third and fourth electrodes, the fifth and sixth electrodes, and so on. In another example, the electrodes may be sequentially energized as pairs by energizing, in sequence, the first and second electrodes, the second and third electrodes, the third and fourth electrodes, and so on. In yet another example, the electrodes may be sequentially energized as pairs by energizing, in sequence, the first and third electrodes, the second and fourth electrodes, the third and fifth electrodes, and so on. Additional systems and methods for sequentially energizing electrodes of an electrode assembly are described, for example, in U.S. Provisional Patent Application Serial No. 63/109,520, filed November 4, 2020. Sequential energization may be used in both monopolar and bipolar configurations.

In the exemplary embodiment, electroporation generator 26, sometimes also referred to herein as a DC energy source, is a biphasic electroporation generator 26 configured to generate a series of DC pulses with alternating polarities - i.e., consecutive DC pulses that produce current in alternating directions. In other embodiments, electroporation generator is a monophasic or polyphasic electroporation generator. In some embodiments, electroporation generator 26 is configured to output energy in DC pulses at selectable energy levels, such as fifty joules, one-hundred joules, two-hundred joules, and the like. Other embodiments may have more or fewer energy settings, and the values of the available settings may be the same or different. In some embodiments, electroporation generator 26 outputs or generates a DC pulse having a peak magnitude of between about 500 V and about 3.5 kV, between about 600 V and 2.5 kV, between about 800 V and about 3.5 kV, between about 600 V and about 2.0 kV, between about 800 V and about 2.5 kV, between about 1.0 kV and about 3.5 kV, between about 600 V and about 1.5 kV, between about 800 V and about 2.0 kV, or between about 1.0 kV and about 2.5 kV. Other embodiments may output or generate any other suitable voltage.

A variable impedance 27 allows the impedance of the system to be varied. Moreover, variable impedance 27 may be used to change one or more characteristics, such as amplitude, duration, pulse shape, and the like, of an output of electroporation generator 26. Although illustrated as a separate component, variable impedance 27 may be incorporated in catheter 14 or generator 26. Variable impedance 27 includes one or more impedance elements, such as resistors, capacitors, or inductors (not shown) connected in series, parallel, or combinations of series and/or parallel. In the illustrated embodiment, variable impedance 27 is connected in series with catheter 14. Alternatively, the impedance elements of variable impedance 27 may be connected in parallel with catheter 14 or in a combination of series and parallel with catheter 14. Moreover, in other embodiments, the impedance elements of variable impedance 27 are connected in series and/or parallel with return electrode 18. Some embodiments include more than one variable impedance 27, each of which may include one or more impedance elements. In such embodiments, each variable impedance 27 may be connected to a different catheter electrode or group of catheter electrodes to allow the impedance through each catheter electrode or group of catheter electrodes to be independently varied. In other embodiments, the impedance of system 10 may not need to be varied and variable impedance 27 may be omitted.

Electroporation generator 26 is configured to generate and supply a pulse signal to electrodes of electrode assembly 12 configured to reduce, minimize, or prevent undesirable or unintended effects of IRE. For example, previous IRE therapy systems may cause skeletal muscle contractions due to the application of high-amplitude, short-duration DC electrical (IRE) pulses. Such skeletal muscle contractions are generally undesirable, for example, because they can render electroanatomical maps (e.g., collected prior to IRE therapy) inaccurate by shifting a patient's body. Additionally, previous IRE therapy systems may generate undesirable gasses within a patient, for example, at the electrodes.

Pulse signals generated by electroporation generator 26 are specifically shaped (e.g., by controlling the phases, amplitude, and pulse duration) to prevent activation of skeletal muscles and nerves (e.g., Phrenic nerve), as well as the myocardium. By avoiding activation of the myocardium, the pulse signals generated by electroporation generator do not have to be timed or gated based on the cardiac cycle or rhythm (e.g., along the R-wave). More specifically, pulse signals generated by electroporation generator 26 are shaped to have a pulse duration and voltage amplitude below the strength-duration curve associated with nerve stimulation or muscle activation. The pulse signals generated by electroporation generator 26 are relatively high strength (i.e., voltage) and frequency (i.e., short pulse duration). By way of example, pulse signals generated by electroporation generator 26 can have a voltage amplitude in the range of 500 V to 3.5 kV, 600 V to 2.5 kV, 800 V to 3.5 kV, 600 V to 2.0 kV, 800 V to 2.5 kV, 1.0 kV to 3.5 kV, 600 V to 1.5 kV, 800 V to 2.0 kV, or 1.0 kV to 2.5 kV, and a pulse duration in the range of 1 nanosecond to 100 microseconds (µs), 1 nanosecond to 50 µs, 0.1 µs to 100 µs, 1 nanosecond to 20 µs, 0.1 µs to 50 µs, 1 µs to 100 µs, 1 nanosecond to 15 µs, 0.1 µs to 20 µs, 0.5 µs to 50 µs, 1 nanosecond to 10 µs, 0.1 µs to 15 µs, 1 nanosecond to 5 µs, 0.1 µs to 10 µs, 0.1 µs to 5 µs, less than 5 µs, less than 4 µs, less than 3 µs, and less than 2 µs. In other embodiments, pulse signals generated by electroporation generator 26 can have a voltage amplitude less than 500 V or greater than 3.5 kV, and can have a pulse duration greater than 100 µs or less than 1 nanosecond. In general, the voltage amplitude and pulse duration needed for efficacious IRE (e.g., to produce continuous lesions) are inversely related, and are selected within a voltage and duration range to avoid nerve stimulation or muscle activation.

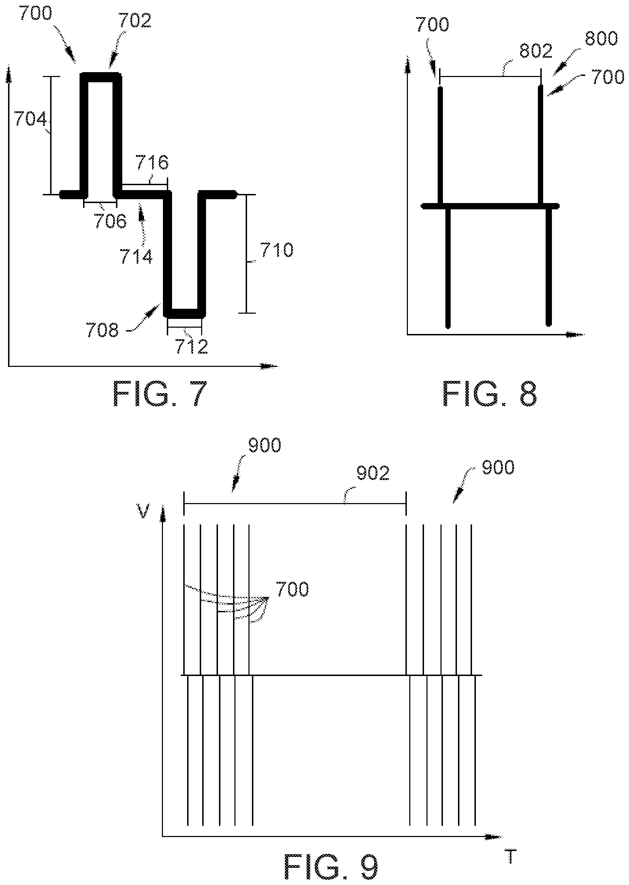

FIG. 7 is a plot illustrating an exemplary pulse signal 700 generated by electroporation generator 26, wherein the vertical axis represents voltage (V) and the horizontal axis represents time (T). As shown in FIG. 7, pulse signal 700 is a biphasic pulse signal including a first phase 702 having a first voltage amplitude 704 with a first polarity and a first pulse duration 706, and a second phase 708 having a second voltage amplitude 710 with a second polarity opposite to the first polarity and a second pulse duration 712. The illustrated pulse signal 700 also includes a third, zero-voltage phase 714 (i.e., pulse signal 700 has a voltage output of zero) separating second phase 708 from first phase 702 such that second phase 708 is generated at a non-zero interval 716 following first phase 702. In other embodiments, pulse signal 700 may not include third phase 714 such that second phase 708 is generated immediately following first phase 702.

In the illustrated embodiment, first phase 702 has a positive voltage, and second phase 708 has a negative voltage. In other embodiments, first phase 702 may have a negative voltage, and second phase 708 may have a positive voltage. Additionally, while pulse signal 700 is shown and described as a biphasic signal in the illustrated embodiment, pulse signal 700 may be other than a biphasic signal in other embodiments (e.g., pulse signal 700 may be monophasic or polyphasic).

Further, in the illustrated embodiment, first phase 702 and second phase 708 have the same voltage amplitude or magnitude, and the same pulse duration (i.e., first pulse duration 706 is equal to second pulse duration 712). That is, pulse signal 700 is a symmetric pulse signal. In other embodiments, first phase 702 may have a different voltage amplitude or magnitude and/or pulse duration than second phase 708 such that pulse signal 700 is asymmetric.

First phase 702 and second phase 708 may generally have any suitable voltage amplitude and pulse duration sufficient to cause ablation by IRE while at the same time avoiding skeletal muscle stimulation. In some embodiments, for example, each of first voltage amplitude 704 and second voltage amplitude 710 is in the range of 500 V to 3.5 kV, 600 V to 2.5 kV, 800 V to 3.5 kV, 600 V to 2.0 kV, 800 V to 2.5 kV, 1.0 kV to 3.5 kV, 600 V to 1.5 kV, 800 V to 2.0 kV, or 1.0 kV to 2.5 kV, and each of first pulse duration 706 and second pulse duration 712 is in the range of 1 nanosecond to 100 µs, 1 nanosecond to 50 µs, 0.1 µs to 100 µs, 1 nanosecond to 20 µs, 0.1 µs to 50 µs, 1 µs to 100 µs, 1 nanosecond to 15 µs, 0.1 µs to 20 µs, 0.5 µs to 50 µs, 1 nanosecond to 10 µs, 0.1 µs to 15 µs, 1 nanosecond to 5 µs, 0.1 µs to 10 µs, 0.1 µs to 5 µs, less than 5 µs, less than 4 µs, less than 3 µs, and less than 2 µs. In one particular embodiment, each of first voltage amplitude 704 and second voltage amplitude 710 is about 1.0 kV, and each of first pulse duration 706 and second pulse duration 712 is about 2 µs. In another particular embodiment, each of first voltage amplitude 704 and second voltage amplitude 710 is about 1.4 kV, and each of first pulse duration 706 and second pulse duration 712 is about 2 µs.

Interval 716 of third phase 714 is generally selected to be of sufficient duration to prevent or avoid first phase 702 and second phase 708 of pulse signal 700 from activating or stimulating skeletal muscle. Interval 716 is generally less than 50 µs, and can be, for example and without limitation, less than 30 µs, less than 20 µs, less than 15 µs, less than 10 µs, less than 5 µs, less than 4 µs, less than 3 µs, less than 2 µs, and even less than 1 µs. In the illustrated embodiment, interval 716 is about 2.5 µs. In another particular embodiment, interval 716 is about 2 µs.

In some embodiments, the voltage amplitude of pulse signal 700 (i.e., first phase 702 and second phase 708) may be selected based on the type of electrode assembly 12 used in system 10. More specifically, certain types of electrode assemblies may be rated for higher voltages than other electrode assemblies, and pulse signal 700 may be tuned accordingly. In some embodiments, for example, pulse signal 700 (i.e., first phase 702 and second phase 708) may have a higher voltage amplitude when used with a loop-type electrode assembly (e.g., electrode loop assembly 200 shown in FIGS. 2 and 3) as compared to a basket-type electrode assembly (e.g., basket electrode assembly 400 shown in FIG. 4). In one particular embodiment, voltage amplitude of pulse signal 700 is in the range of 1 kV to 2.5 kV when used with a loop-type electrode assembly, and in the range of 800 V to 1.5 kV when used with a basket-type electrode assembly.

Characteristics of the pulse signal 700 (e.g., voltage amplitude, first and second pulse durations 706, 712, etc.) may also be tuned according to whether the electrode assembly 12 is configured as a monopolar electrode assembly or a bipolar electrode assembly. For example, skeletal muscle recruitment may be more prominent in monopolar IRE as compared to bipolar IRE. Accordingly, the pulse signal used in monopolar IRE may have narrower or more stringent ranges of acceptable pulse characteristics (e.g., voltage amplitude and pulse durations) as compared to bipolar IRE.

In one example, a loop-type electrode assembly (e.g., electrode loop assembly 200 shown in FIGS. 2 and 3) configured as a monopolar electrode assembly may utilize a pulse signal 700 having a voltage amplitude in the range of 500 V to 3.5 kV, or in the range of 800 V to 3.0 kV, and first and second pulse durations 706 and 712 in the range of 0.5 µs to 3.0 µs, or in the range of 0.5 µs to 1.5 µs. A loop-type electrode assembly (e.g., electrode loop assembly 200 shown in FIGS. 2 and 3) configured as a bipolar electrode assembly may utilize a pulse signal 700 having a voltage amplitude in the range of 500 V to 3.5 kV, or in the range of 600 V to 1.4 kV, and first and second pulse durations 706 and 712 in the range of 0.5 µs to 3 µs, or in the range of 1 µs to 1.5 µs.

In another example, a basket-type electrode assembly (e.g., basket electrode assembly 400 shown in FIG. 4) configured as a monopolar electrode assembly may utilize a pulse signal 700 having a voltage amplitude in the range of 500 V to 3.5 kV, and first and second pulse durations 706 and 712 in the range of 0.5 µs to 3 µs. A basket-type electrode assembly (e.g., basket electrode assembly 400 shown in FIG. 4) configured as a bipolar electrode assembly may utilize a pulse signal 700 having a voltage amplitude in the range of 500 V to 3.5 kV, and first and second pulse durations 706 and 712 in the range of 0.5 µs to 3 µs.

In another example, a grid electrode assembly (e.g., grid electrode assembly 500 shown in FIG. 5) configured as a monopolar electrode assembly may utilize a pulse signal 700 having a voltage amplitude in the range of 500 V to 3.5 kV, and first and second pulse durations 706 and 712 in the range of 0.5 µs to 3 µs. A grid electrode assembly (e.g., grid electrode assembly 500 shown in FIG. 5) configured as a bipolar electrode assembly may utilize a pulse signal 700 having a voltage amplitude in the range of 500 V to 3.5 kV, and first and second pulse durations 706 and 712 in the range of 0.5 µs to 3 µs.

In yet another example, an expandable electrode assembly (e.g., expandable electrode assembly 600 shown in FIG. 6) configured as a bipolar electrode assembly may utilize a pulse signal 700 having a voltage amplitude in the range of 500 V to 3.5 kV, in the range of 500 V to 2.5 kV, the range of 600 V to 3.0 kV, in the range of 600 V to 2.5 kV, or in the range of 800 V to 2.5 kV, and first and second pulse durations 706 and 712 in the range of 400 nanoseconds to 20 µs, or in the range of 500 nanoseconds to 1.5 µs. Additionally, the pulse signal 700 used for an expandable electrode assembly (e.g., expandable electrode assembly 600 shown in FIG. 6) may have an interval 716 between the first phase 702 and the second phase 708 in the range of 350 nanoseconds to 1 ms, or in the range of 500 nanoseconds to 1.5 µs.

Electroporation generator 26 may generate pulse signal 700 in a repeating pattern such that a plurality of pulse signals 700 are generated and applied to electrodes of electrode assembly 12 at a repeating pulse period. Such plurality of pulse signals 700 are collectively referred to herein as a burst signal.

FIG. 8 illustrates an example burst signal 800 including two pulse signals 700 generated at a pulse period 802. Pulse period 802 may be any suitable period that enables system 10 to function as described herein. By way of example, pulse period 802 may be in the range of 0.5 milliseconds (ms) to 50 ms, 1 ms to 50 ms, 0.5 ms to 30 ms, 1 ms to 30 ms, 0.5 ms to 25 ms, 0.5 ms to 20 ms, 1 ms to 25 ms, 0.5 ms to 10 ms, 1 ms to 20 ms, 1 ms to 10 ms, 0.5 ms to 5 ms, and 1 ms to 5 ms. In the illustrated embodiment, pulse period 802 is about 4 ms. In some embodiments, pulse period 802 is selected to allow a power supply (e.g., a capacitor) of electroporation generator 26 to recharge to a sufficient voltage to maintain the voltage amplitude of pulse signal 700 at or near the target voltage amplitude (e.g., at least 90% of a target voltage amplitude).

In some embodiments, electroporation generator 26 may generate burst signals in a repeating pattern such that a plurality of burst signals are generated and applied to electrodes of electrode assembly 12 at a repeating period, referred to as a burst period. FIG. 9, for example, illustrates an example burst signal 900 generated at a repeating burst period 902. Burst period 902 may be any suitable period that enables system 10 to function as described herein. By way of example, burst period 902 may be in the range of 50 ms to 5 seconds (s), 100 ms to 5 s, 100 ms to 2 s, 100 ms to 1 s, 200 ms to 1 s, 200 ms to 800 ms, and 300 ms to 700 ms. In the illustrated embodiment, burst period 902 is about 500 ms. In some embodiments, burst period 902 is selected to allow a power supply (e.g., a capacitor) of electroporation generator 26 to recharge to a sufficient voltage to maintain the voltage amplitude of pulse signal 700 at or near the target voltage amplitude (e.g., at least 90% of a target voltage amplitude). Additionally or alternatively, burst period 902 may vary based on a patient's cardiac cycle or rhythm. In one embodiment, for example, the burst signal 900 is generated according to an R-wave gating strategy (e.g., in sync with a patient's heart rate). In such embodiments, the burst signal 900 may be generated in response to or triggered based on detection of an R-wave in an electrocardiogram. In such embodiments, the burst period 902 may be variable (i.e., not fixed or constant). For example, the burst period 902 may vary within the range of 500 ms to 1.5 s, within the range of 600 ms and 1.2 s, or within any other suitable range that enables system 10 to function as described herein. Such burst periods and gating strategies may be used in connection with any of the electrode assemblies described herein.

The plot illustrated in FIG. 9 includes two burst signals 900, though it should be understood that electroporation generator 26 may generate more than two burst signals 900. By way of example, electroporation generator 26 may generate a series of burst signals 900 (i.e., at a repeating burst period 902) that includes at least 5 burst signals, at least 10 burst signals, at least 20 burst signals, at least 30 burst signals, at least 40 burst signals, at least 50 burst signals, at least 75 burst signals, at least 100 burst signals, or up to 200 burst signals. In other embodiments, electroporation generator 26 may generate a series of more than 200 burst signals at a repeating burst period.

Further, in the illustrated embodiment, burst signal 900 includes 5 pulse signals 700, although it should be understood that burst signal 900 may include any suitable number of pulse signals 700 that enables system 10 to function as described herein. By way of example, burst signal 900 can include at least 10 pulse signals, at least 15 pulse signals, at least 20 pulse signals, at least 30 pulse signals, at least 40 pulse signals, at least 50 pulse signals, at least 75 pulse signals, at least 100 pulse signals, at least 150 pulse signals, or up to 200 pulse signals. In other embodiments, burst signal 900 may include less than 5 pulse signals 700 or greater than 200 pulse signals 700. In one particular embodiment, burst signal 900 includes 50 pulse signals 700, and has a burst period 902 of 0.5 s. In another particular embodiment, burst signal 900 includes 1,000 pulse signals 700, and has a burst period 902 of 0.5 s. Such burst signals and burst periods are suitable for use with any of the electrode assemblies described herein.

The relatively high voltages and short pulse durations of the pulse signals generated by electroporation generator 26 may result in significant electromagnetic interference (EMI), or noise, being introduced into electroporation generator 26, its components, catheter 14, or tissue 16 of the patient, and potentially adversely affecting operation of system 10. Accordingly, embodiments of the present disclosure include certain features to reduce sources of noise and to mitigate the effects of the high frequency high voltage switching within electroporation generator 26 for the purpose of producing the high-amplitude short duration pulse signals.

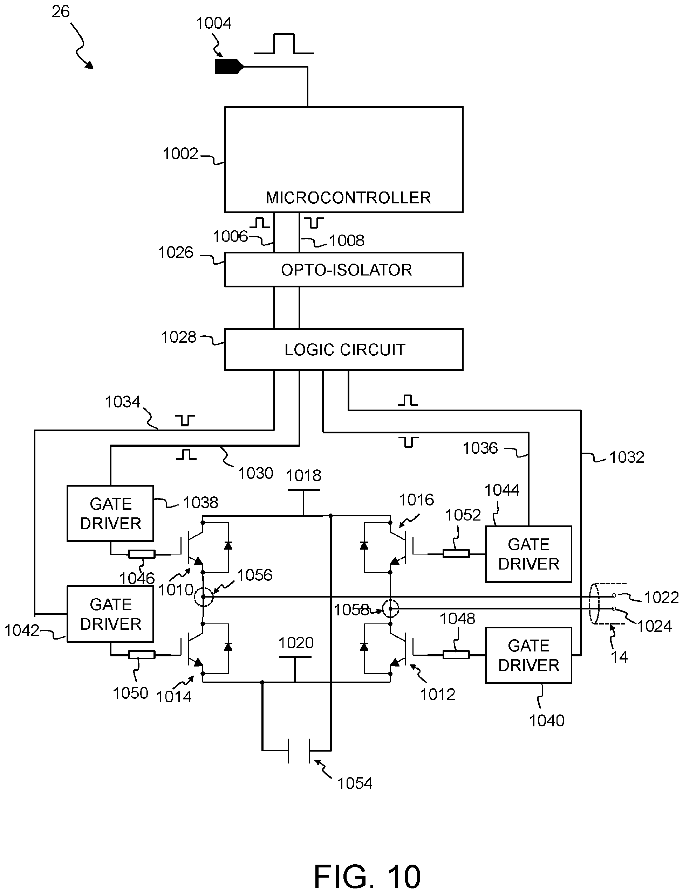

For example, FIG. 10 is a schematic diagram of exemplary electroporation generator 26. Electroporation generator 26 includes a microcontroller 1002 or other programmable processing device that controls generation of the pulse signals in response to a trigger signal 1004. Trigger signal 1004 may be generated internal to electroporation generator 26 or externally by another system. In certain embodiments, trigger signal is a discrete logic-level DC signal supplied to microcontroller 1002 as a result of, for example, an at least momentary closure of a switching circuit by a switch or button actuated by a user. In response to trigger signal 1004, microcontroller 1002 initiates a single pulse, a burst of pulses, or a plurality of bursts, for example.

Microcontroller 1002, in generating a single pulse signal, generates a first pulse control signal 1006 and a second pulse control signal 1008 for the purpose of controlling a plurality of semiconductor switches. First pulse control signal 1006 and second pulse control signal 1008 are logic level DC signals generated by microcontroller 1002. The semiconductor switches may be any suitable power semiconductor capable of a high-voltage standoff, high current conduction, and operable at a high frequency, such as an insulated-gate bipolar transistor (IGBT). In the embodiment of FIG. 10, the semiconductor switches are implemented as IGBTs 1010, 1012, 1014, 1016 connected in a bridge configuration to regulate application of positive high voltage DC (+HVDC) supply 1018 and negative high voltage DC (-HVDC) supply 1020 to catheter 14 and, more specifically, first and second conductors 1022 and 1024 that deliver the pulse signal to electrode assembly 12. Microcontroller 1002, in generating a burst of pulse signals, controls IGBTs 1010, 1012, 1014, 1016 to commutate at a high frequency (e.g., about 500 kilohertz) to produce each pulse signal.

Microcontroller 1002 is communicatively coupled and electrically isolated from IGBTs 1010, 1012, 1014, 1016 by an opto-isolator 1026. Opto-isolator 1026, also referred to as an opto-coupler, prevents, for example, noise generated by high frequency switching of IGBTs 1010, 1012, 1014, 1016 from reaching microcontroller 1002. Opto-isolator 1026 relays first pulse control signal 1006 and second pulse control signal 1008 from microcontroller 1002 to a logic circuit 1028 that translates the two logic level DC signals into four gate driving signals 1030, 1032, 1034, 1036. Logic circuit 1028 derives each of gate driving signals 1030, 1032, 1034, 1036 from first pulse control signal 1006 and second pulse control signal 1008, and ensures that gate driving signals 1030, 1032, 1034, 1036 do not connect, or short, the opposite-polarity HVDC supplies (+HVDC supply 1018 and -HVDC supply 1020), for example, momentarily during a transition from a +HVDC phase to a -HVDC phase of the pulse signal. For example, in certain embodiments, logic circuit 1028 derives gate driving signals 1034 and 1036 as inversions of gate driving signals 1030 and 1032.

Generally, in at least some embodiments, microcontroller 1002 does not source sufficient current to drive the gates of IGBTs 1010, 1012, 1014, 1016. Gate current for power semiconductor switches typically rises with high voltage and high current capacity. Accordingly, electroporation generator 26 includes gate drivers 1038, 1040, 1042, 1044 for operating IGBTs 1010, 1012, 1014, 1016, respectively. Gate drivers 1038, 1040, 1042, 1044 further isolate microcontroller 1002 and other aspects of the digital circuit from the high-voltage high-current portions of electroporation generator 26. Gate drivers 1038, 1040, 1042, 1044 control commutation of IGBTs 1010, 1012, 1014, 1016 according to gate driving signals 1030, 1032, 1034, 1036. Gate drivers 1038, 1040, 1042, 1044 drive gates of IGBTs 1010, 1012, 1014, 1016 through gate driving impedances 1046, 1048, 1050, 1052. Gate driving impedances 1046, 1048, 1050, 1052 are selected both to produce a sufficient current rise through IGBTs 1010, 1012, 1014, 1016 and to avoid oscillatory responses by IGBTs 1010, 1012, 1014, 1016. In certain embodiments, gate driving impedances 1046, 1048, 1050, 1052 are resistors in the range of 6-8 ohms. In at least some embodiments, gate driving impedances 1046, 1048, 1050, 1052 are 6.8 ohm resistors.

Electroporation generator 26 may be implemented, in certain embodiments, on one or more printed circuit boards (PCBs) on which microcontroller 1002, opto-isolator 1026, logic circuit 1028, gate drivers 1038, 1040, 1042, 1044, and IGBTs 1010, 1012, 1014, 1016 may be disposed. At least some traces on the PCB conduct high-voltage DC that is switched at a high frequency. For example, traces connecting +HVDC supply 1018 to IGBTs 1010, 1012, 1014, 1016, and traces supplying current from IGBTs 1010, 1012, 1014, 1016 to terminals 1056 and 1058 for first and second conductors 1022 and 1024 each carry the pulse signal generated by high-frequency switching of IGBTs 1010, 1012, 1014, 1016, and thus are susceptible to introducing noise to electroporation generator 26. In certain embodiments, such traces should be sufficiently wide and as short as possible to reduce the introduction of noise resulting from the periodic high di/dt conditions on those traces. In certain embodiments, these traces should be at least 0.12 inches wide. Likewise, at least some traces conduct significant amounts of high-frequency switched current for the purpose of driving gates of IGBTs 1010, 1012, 1014, 1016. For example, traces extending between gate drivers 1038, 1040, 1042, 1044 and their respective IGBTs 1010, 1012, 1014, 1016 are each also susceptible to introducing noise resulting from high di/dt conditions on those traces. Accordingly, those traces should also be sufficiently wide and as short as possible to reduce the introduction of noise. In certain embodiments, for example, the traces between gate drivers 1038, 1040, 1042, 1044 and their respective IGBTs 1010, 1012, 1014, 1016 should be at least 0.06 inches wide.

In certain embodiments, electroporation generator 26 includes additional components between each of gate drivers 1038, 1040, 1042, 1044 and IGBTs 1010, 1012, 1014, 1016. For example, in certain embodiments, one or more capacitors are coupled in parallel with the gate of the semiconductor switch to function as a current supply for driving that gate. In certain embodiments, one or more diodes are coupled in parallel with the gate of the semiconductor switch to function, for example, as transient voltage suppression or as current blocking devices. In certain embodiments, one or more EMI suppression devices are coupled to the gate driving branch to mitigate noise originating from, for example, high frequency switching of IGBTs 1010, 1012, 1014, 1016.

In certain embodiments, electroporation generator 26 includes one or more impedance matching circuits (not shown) connected in series with the high-voltage DC output, i.e., in series with first and second conductors 1022 and 1024. The impedance matching circuits mitigate impedance discontinuities that may occur or that may be inherent at various portions of the high-voltage DC transmission line formed by the traces or other conductors between +HVDC supply 1018, -HVDC supply 1020, and electrode assembly 12 of catheter 14. For example, an impedance discontinuity may exist where catheter 14 connects to electroporation generator 26, which may result in signal reflections within electroporation generator 26 that ultimately manifest as noise and losses in system 10.

+HVDC supply 1018 and -HVDC supply 1020 are opposite polarity DC voltage levels. For example, in certain embodiments, +HVDC supply 1018 may be at a potential of about 3500 VDC and -HVDC supply 1020 may be at a potential of about zero, or effectively ground. Electroporation generator 26, in at least some embodiments, includes a high-voltage capacitor 1054 to function as the high voltage current source for electrode assembly 12. A single pulse signal includes a first phase having a first polarity high voltage, a second phase having about potential of zero volts, and a third phase having a second polarity high voltage. Catheter 14 supplies the high voltage pulse signal over first and second conductors 1022 and 1024. Accordingly, the polarity of the high voltage pulse signal can be switched by alternatingly applying, in time, +HVDC supply 1018 to first conductor 1022 and second conductor 1024, and -HVDC supply 1020 to second conductor 1024 and first conductor 1022. Likewise, 0 VDC is achieved by disconnecting both first and second conductors 1022 and 1024 and allowing their potential to float. Consequently, due to the conductive properties of a blood/saline solution in the body 17 of the patient, there should be no potential between electrodes of electrode assembly 12, and thus no potential between first and second conductors 1022 and 1024.

For example, where +HVDC supply 1018 is at a potential of about 3500 VDC and -HVDC supply 1020 is at a potential of about 0 VDC, the first phase of the pulse signal is produced by closing IGBT 1010 and opening IGBT 1014 to apply +HVDC supply 1018 to first conductor 1022; and closing IGBT 1012 and opening IGBT 1016 to apply - HVDC supply 1020 to second conductor 1024, thereby producing a +3500 VDC signal for a first duration. After the first duration, IGBT 1010 and IGBT 1012 are opened to allow the potential of first and second conductors 1022 and 1024 to float, thereby producing 0 VDC for the second phase of the pulse signal for a second duration. After the second duration, IGBT 1014 is closed to apply -HVDC supply 1020 to first conductor 1022, and IGBT 1016 is closed to apply +HVDC supply 1018 to second conductor 1024, thereby producing a - 3500 VDC signal for a third duration. After the third duration, IGBT 1014 and IGBT 1016 are opened to return the potential across first and second conductors 1022 and 1024 to 0 VDC.

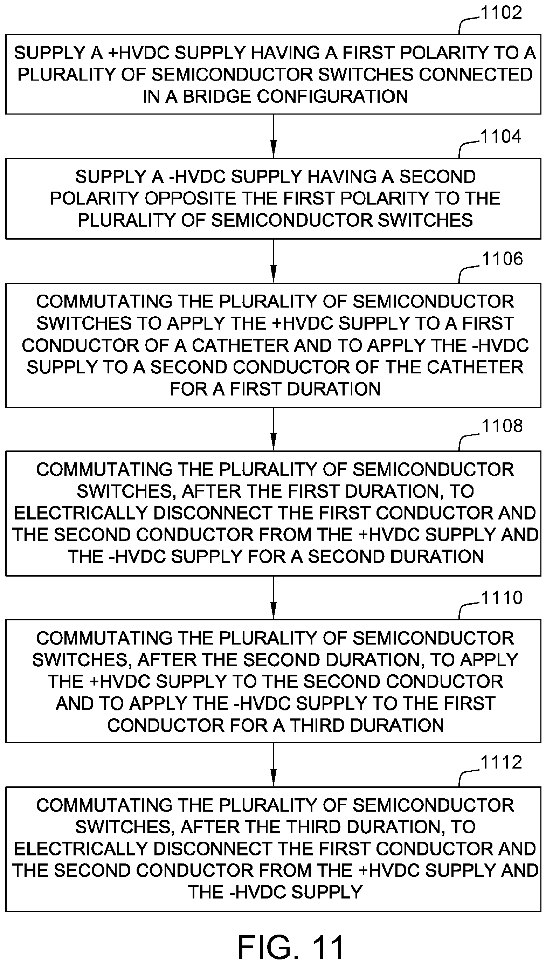

FIG. 11 is a flow diagram of an example method 1100 of generating a pulse signal. Referring to FIGS. 10 and 11, the method includes supplying 1102 +HVDC supply 1018 having a first polarity to a plurality of semiconductor switches, such as IGBTs 1010, 1012, 1014, 1016 connected in a bridge configuration. -HVDC supply 1020, having a second polarity opposite the first polarity, is supplied 1104 to the plurality of semiconductors. To generate the biphasic pulse signal, microcontroller 1002 controls commutation of IGBTs 1010, 1012, 1014, 1016. More specifically, the plurality of semiconductors are each commutated 1106 to apply +HVDC supply 1018 to first conductor 1022 of catheter 14 and to apply the -HVDC supply 1020 to second conductor 1024 of catheter 14 for a first duration. The plurality of semiconductors are each then commutated 1108, after the first duration, to electrically disconnect first conductor 1022 and second conductor 1024 from +HVDC supply 1018 and -HVDC supply 1020 for a second duration. The plurality of semiconductors are each then commutated 1110, after the second duration, to apply +HVDC supply 1018 to second conductor 1024 and to apply - HVDC supply 1020 to first conductor 1022 for a third duration. The plurality of semiconductors are each then commutated 1112, after the third duration, to electrically disconnect first and second conductors 1022 and 1024 from +HVDC supply 1018 and - HVDC supply 1020.

Although certain steps of the example method are numbered, such numbering does not indicate that the steps must be performed in the order listed. Thus, particular steps need not be performed in the exact order they are presented, unless the description thereof specifically require such order. The steps may be performed in the order listed, or in another suitable order.

Although the embodiments and examples disclosed herein have been described with reference to particular embodiments, it is to be understood that these embodiments and examples are merely illustrative of the principles and applications of the present disclosure. It is therefore to be understood that numerous modifications can be made to the illustrative embodiments and examples and that other arrangements can be devised without departing from the spirit and scope of the present disclosure. Thus, it is intended that the present description cover the modifications and variations of these embodiments and their equivalents.

The present invention is set out in the claims that follow.

## Claims

1. An electroporation system comprising:
a catheter shaft (44);
an electrode assembly (200) coupled to the catheter shaft at a distal end thereof, the electrode assembly including at least one electrode, wherein the electrode assembly is configured as one of an electrode loop assembly, and a planar electrode assembly; and
an electroporation generator (26) coupled in communication with the at least one electrode, the electroporation generator (26) configured to supply a biphasic pulse (700) signal to the at least one electrode, the biphasic pulse signal (700) comprising:
a first phase (702) having a first polarity and a first pulse duration; and
a second phase (708) having a second polarity opposite to the first polarity, and a second pulse duration, each of the first phase (702) and second phase (708) having a voltage amplitude of at least 500 volts and a pulse duration of less than 20 microseconds,
wherein the second phase (708) is generated at a non-zero interval following the first phase (702), and
wherein the electroporation generator (26) comprises:
a microcontroller (402);
a plurality of switching elements (410, 412, 414, 416); and
an opto-isolator (1026) communicatively coupling the microcontroller (402) with each of the plurality of switching elements and configured to prevent noise generated by switching of the plurality of switching elements from reaching the microcontroller.

2. The electroporation system of claim 1, wherein each of the first and second phase has a voltage amplitude in the range of 500 V to 3.5 kV, 600 V to 2.5 kV, 800 V to 3.5 kV, 600 V to 2.0 kV, 800 V to 2.5 kV, 1.0 kV to 3.5 kV, 600 V to 1.5 kV, 800 V to 2.0 kV, or 1.0 kV to2.5 kV and a pulse duration in the range of 1 nanosecond to 20 microseconds, or
a pulse duration in the range of 0,1 microseconds to 20 microseconds, or
a pulse duration in the range of 1 nanosecond to 10 microseconds, or
a pulse duration in the range of 0,1 microseconds to 15 microseconds, or
a pulse duration in the range of 1 nanosecond to 5 microseconds, or
a pulse duration in the range of 0,1 microseconds to 10 microseconds, or
a pulse duration in the range of 0,1 microseconds to 5 microseconds, or
a pulse duration of less than 1.5 microseconds, or
a pulse duration of less than 5 microseconds, or
a pulse duration of less than 4 microseconds, or
a pulse duration of less than 3 microseconds, or
a pulse duration of less than 2 microseconds, or
a pulse duration of less than 1 nanosecond, or
wherein each of the first pulse duration, the second pulse duration, and the non-zero interval is less than 3 microseconds, or wherein each of the first pulse duration, the second pulse duration, and the non-zero interval is less than 3 microseconds and each of the first phase and second phase has a voltage amplitude of at least 600 volts.

3. The electroporation system of claim 1 or 2, wherein the electroporation generator (26) is further configured to generate a plurality of the biphasic pulse signals, wherein each of the plurality of biphasic pulse signals is generated at a pulse period in a range of 0.5 milliseconds to 10 milliseconds.

4. The electroporation system of claim 1, wherein the electrode assembly is configured as a bipolar electrode loop assembly, and wherein each of the first and second phase has a voltage amplitude of at least 600 volts and a pulse duration of less than 3 microseconds.

5. The electroporation system of claim 4, wherein each of the first and second phase has a voltage amplitude in the range of 600 volts to 1.4 kV, and a pulse duration in the range of 1 microsecond to 1.5 microseconds.

6. The electroporation system of claim 1, wherein the electrode assembly is configured as a monopolar electrode loop assembly, and wherein each of the first and second phase has a voltage amplitude of at least 800 volts and a pulse duration of less than 3 microseconds.

7. The electroporation system of claim 6, wherein each of the first and second phase has a voltage amplitude in the range of 800 volts to 3.0 kV, and a pulse duration in the range of 0.5 microseconds to 1.5 microseconds.

8. The electroporation system of claim 1, wherein the electrode assembly (200) is configured as a bipolar expandable electrode assembly comprising an expandable isolation member, and wherein each of the first and second phase has a voltage amplitude of at least 500 volts and a pulse duration of less than 20 microseconds.

9. The electroporation system of claim 8, wherein each of the first and second phase has a voltage amplitude in the range of 600 volts to 2.5 kV, and a pulse duration in the range of 500 nanoseconds to 1.5 microseconds.

10. An electroporation generator (26) comprising:
a positive high-voltage direct current (+HVDC) supply (1008) having a first polarity;
a negative high-voltage direct current (-HVDC) supply (1012) having a second polarity opposite the first polarity;
a plurality of semiconductor switches (410, 412, 414, 416) connected in a bridge configuration to regulate application of the +HVDC supply (1008) and the -HVDC supply (1012) to first and second conductors (422, 424) for a catheter (14); and
a microcontroller (402) communicatively coupled to the plurality of semiconductor switches (410, 412, 414, 416) and configured to control commutation of the plurality of semiconductor switches (410, 412, 414, 416) to transmit a biphasic pulse signal (500) through the first and second conductors (422, 424) for the catheter (14), wherein the microcontroller (402) is further configured to generate the biphasic pulse signal (500) comprising:
a first phase having the first polarity and a first pulse duration;
a second phase having the second polarity, and a second pulse duration, each of the first and second phase having a voltage amplitude of at least 500 volts and a pulse duration of less than 20 microseconds; and
wherein the second phase is generated at a non-zero interval following the first phase; and
an opto-isolator (1026) communicatively coupling the microcontroller (402) communicatively coupling the microcontroller (402) with each of the plurality of semiconductor switches and configured to prevent noise generated by switching of the plurality of semiconductor switches from reaching the microcontroller.

11. The electroporation generator (26) according to claim 10, wherein the plurality of semiconductor switches (410, 412, 414, 416) comprises a plurality of insulated-gate bipolar transistors.

12. The electroporation generator of claim 10 further comprising a plurality of gate drivers (438, 440, 442, 444) coupled between the microcontroller and corresponding ones of the plurality of semiconductor switches (410, 412, 414, 416), wherein the plurality of gate drivers (438, 440, 442, 444) are configured to supply gate driving current based on at least one signal from the microcontroller (402).

13. The electroporation generator of claim 12 further comprising a gate driving impedance coupled between each of the plurality of gate drivers and the corresponding ones of the plurality of semiconductor switches, the gate driving impedance configured to calibrate a rise time for high-voltage DC supplied through the plurality of semiconductor switches, and further configured to reduce oscillatory response by the plurality of semiconductor switches.

14. A method of generating a pulse signal, the method comprising:
supplying a positive high-voltage direct current (+HVDC) supply (1008) having a first polarity and having a voltage amplitude of at least 500 volts to a plurality of semiconductor switches (410, 412, 414, 416) connected in a bridge configuration;
supplying a negative high-voltage direct current (-HVDC) supply (1012) having a second polarity opposite the first polarity to the plurality of semiconductors (410, 412, 414, 416) and having a voltage amplitude of at least 500 volts;
commutating the plurality of semiconductor switches (410, 412, 414, 416) to apply the +HVDC supply (1012) to a first conductor (422) of a catheter (14) and to apply the - HVDC supply (1012) to a second conductor (424) of the catheter (14) for a first duration less than 20 microseconds;
commutating the plurality of semiconductor switches (410, 412, 414, 416), after the first duration, to electrically disconnect the first conductor (422) and the second conductor (424) from the +HVDC supply (1008) and the -HVDC supply (1012) for a second non-zero duration;
commutating the plurality of semiconductor switches (410, 412, 414, 416), after the second non-zero duration, to apply the +HVDC supply (1008) to the second conductor (424) and to apply the -HVDC supply (1012) to the first conductor (422) for a third duration of less than 20 microseconds;
commutating the plurality of semiconductor switches (410, 412, 414, 416), after the third duration, to electrically disconnect the first conductor (422) and the second conductor (424) from the +HVDC supply (1008) and the -HVDC supply (1012), wherein commutating the plurality of semiconductor switches (410, 412, 414, 416) during the first to third durations transmits a biphasic pulse signal (700) through the first and second conductors (422, 424), the biphasic pulse signal comprising a first phase having the first polarity and the first duration and a second phase having the second polarity and the second duration; and
preventing noise generated by commutating the plurality of semiconductor switches (410, 412, 414, 416) from reaching the microcontroller (402) using an opto-isolator (1026) that communicatively couples the microcontroller (402) with each of the plurality of semiconductor switches (410, 412, 414, 416).

## Patentansprüche

1. Elektroporationssystem mit:
einem Katheterschaft (44);
einer Elektrodenanordnung (200), die mit dem Katheterschaft an seinem distalen Ende gekoppelt ist, wobei die Elektrodenanordnung mindestens eine Elektrode aufweist, die Elektrodenanordnung konfiguriert ist als eine von einer Elektrodenschleifenanordnung und einer planaren Elektrodenanordnung; und
einem Elektroporationsgenerator (26), der kommunikativ mit mindestens einer Elektrode gekoppelt ist, wobei der Elektroporationsgenerator (26) konfiguriert ist zum Liefern eines Zweiphasenpulssignals (700) an die mindestens eine Elektrode, wobei das Zweiphasenpulssignal (700) aufweist:
eine erste Phase (702) mit einer ersten Polarität und einer ersten Pulsdauer; und
eine zweite Phase (708) mit einer zweiten Polarität, die zu der ersten Polarität entgegengesetzt ist, und einer zweiten Pulsdauer, wobei jede von der ersten Phase (702) und der zweiten Phase (708) eine Spannungsamplitude von mindestens 500 Volt und eine Pulsdauer von weniger als 20 Mikrosekunden aufweist,
wobei die zweite Phase (708) bei einem Nicht-Null-Intervall, das der ersten Phase (702) folgt, erzeugt wird, und
wobei der Elektroporationsgenerator (26) aufweist:
einen Mikrocontroller (402);
eine Mehrzahl von Schaltelementen (410, 412, 414, 416); und
einen Opto-Isolator (1026), der kommunikativ den Mikrocontroller (402) mit jedem von der Mehrzahl von Schaltelementen koppelt, und der konfiguriert ist zum Verhindern, dass Rauschen, das durch Schalten der Mehrzahl von Schaltelementen erzeugt wird, den Mikrocontroller erreicht.

2. Elektroporationssystem nach Anspruch 1, bei dem jede von der ersten und zweiten Phase eine Spannungsamplitude in dem Bereich von 500 V bis 3,5 KV, 600 V bis 2,5 KV, 800V bis 3,5 KV, 600 V bis 2,0 KV, 800 V bis 2,5 KV, 1,0 kV bis 3,5 KV, 600 V bis 1,5 KV, 800 V bis 2,0 KV oder 1,0 KV bis 2,5 KV und eine Pulsdauer in dem Bereich von 1 Nanosekunde bis 20 Mikrosekunden aufweist, oder
eine Pulsdauer in dem Bereich von 1 Nanosekunden bis 20 Mikrosekunden, oder
eine Pulsdauer in dem Bereich von 0,1 Mikrosekunden bis 20 Mikrosekunden, oder
eine Pulsdauer in dem Bereich von 1 Nanosekunde bis 10 Mikrosekunden, oder
eine Pulsdauer in dem Bereich von 0,1 Mikrosekunden bis 15 Mikrosekunden, oder
eine Pulsdauer in dem Bereich von 1 Nanosekunde bis 5 Mikrosekunden, oder
eine Pulsdauer in dem Bereich von 0,1 Mikrosekunden bis 10 Mikrosekunden, oder
eine Pulsdauer in dem Bereich von 0,1 Mikrosekunden bis 5 Mikrosekunden, oder
eine Pulsdauer von weniger als 1,5 Mikrosekunden, oder
eine Pulsdauer von weniger als 5 Mikrosekunden, oder
eine Pulsdauer von weniger als 4 Mikrosekunden, oder
eine Pulsdauer von weniger als 3 Mikrosekunden, oder
eine Pulsdauer von weniger als 2 Mikrosekunden, oder
eine Pulsdauer von weniger als 1 Nanosekunde, aufweist, oder
wobei jede von der ersten Pulsdauer, der zweiten Pulsdauer und dem Nicht-Null-Intervall kleiner als 3 Mikrosekunden ist, oder jede von der ersten Pulsdauer, der zweiten Pulsdauer und dem Nicht-Null-Intervall kleiner als 3 Mikrosekunden ist und jede von der ersten Phase und der zweiten Phase eine Spannungsamplitude von mindestens 600 Volt aufweist.

3. Elektroporationssystem nach Anspruch 1 oder 2, bei dem der Elektroporationsgenerator (26) ferner konfiguriert ist zum Erzeugen einer Mehrzahl von Zweiphasenpulssignalen, wobei jedes von der Mehrzahl von Zweiphasenpulssignalen bei einer Pulsperiode in einem Bereich von 0,5 Millisekunden bis 10 Millisekunden erzeugt wird.

4. Elektroporationssystem nach Anspruch 1, bei dem die Elektrodenanordnung konfiguriert ist als eine zweiphasige Elektrodenschleifenanordnung, und jede von der ersten und zweiten Phase eine Spannungsamplitude von mindestens 600 Volt und eine Pulsdauer von weniger als 3 Mikrosekunden hat.

5. Elektroporationssystem nach Anspruch 4, bei dem jede von der ersten und zweiten Phase eine Spannungsamplitude in dem Bereich von 600 Volt bis 1,4 KV und eine Pulsdauer in dem Bereich von 1 Mikrosekunde bis 1,5 Mikrosekunden hat.

6. Elektroporationssystem nach Anspruch 1, bei dem die Elektrodenanordnung konfiguriert ist als eine einphasige Elektrodenschleifenanordnung, und jede von der ersten und zweiten Phase eine Spannungsamplitude von mindestens 800 Volt und eine Pulsdauer von weniger als 3 Mikrosekunden hat.

7. Elektroporationssystem nach Anspruch 6, bei dem jede von der ersten und zweiten Phase eine Spannungsamplitude in dem Bereich von 800 Volt bis 3,0 KV und eine Pulsdauer in dem Bereich von 0,5 Mikrosekunden bis 1,5 Mikrosekunden hat.

8. Elektroporationssystem nach Anspruch 1, bei dem die Elektrodenanordnung (200) konfiguriert ist als eine zweiphasige expandierbare Elektrodenanordnung mit einem expandierbaren Isolationselement, und jede von der ersten und zweiten Phase eine Spannungsamplitude von mindestens 500 Volt und eine Pulsdauer von weniger als 20 Mikrosekunden hat.

9. Elektroporationssystem nach Anspruch 8, bei dem jede von der ersten und zweiten Phase eine Spannungsamplitude in dem Bereich von 600 Volt bis 2,5 KV und eine Pulsdauer in dem Bereich von 500 Nanosekunden bis 1,5 Mikrosekunden hat.

10. Elektroporationsgenerator (26) mit:
einer positiven Hochspannungsgleichstrom (+HVDC) Versorgung (1008) mit einer ersten Polarität;
einer negativen Hochspannungsgleichstrom (-HVDC) Versorgung (1012) mit einer zweiten Polarität, die der ersten Polarität entgegengesetzt ist;
einer Mehrzahl von Halbleiterschaltern (410, 412, 414, 416), die in einer Brückenkonfiguration verbunden sind zum Steuern der Anlegung der +HVDC-Versorgung (1008) und der - HVDC-Versorgung (1012) an einen ersten und zweiten Leiter (422, 424) für einen Katheter (14); und
einem Mikrocontroller (402), der kommunikativ mit der Mehrzahl von Halbleiterschaltern (410, 412, 414, 416) gekoppelt und konfiguriert ist zum Steuern der Kommutation der Mehrzahl von Halbleiterschaltern (410, 412, 414, 416), um ein Zweiphasenpulssignal (500) über den ersten und zweiten Leiter (422, 424) für den Katheter (14) zu übertragen, wobei der Mikrocontroller (402) ferner konfiguriert ist zum Erzeugen des Zweiphasenpulssignals (500), das aufweist:
eine erste Phase mit der ersten Polarität und einer ersten Pulsdauer;
eine zweite Phase mit der zweiten Polarität und einer zweiten Pulsdauer, wobei jede von der ersten und zweiten Phase eine Spannungsamplitude von mindestens 500 Volt und eine Pulsdauer von weniger als 20 Mikrosekunden hat; und
wobei die zweite Phase bei einem Nicht-Null-Intervall, das der ersten Phase folgt, erzeugt wird; und
einem Opto-Isolator (1026), der kommunikativ den Mikrocontroller (402) mit jedem von der Mehrzahl von Halbleiterschaltern koppelt und konfiguriert ist zum Verhindern, dass Rauschen, das durch Schalten der Mehrzahl von Halbleiterschaltern erzeugt wird, den Mikrocontroller erreicht.

11. Elektroporationsgenerator (26) nach Anspruch 10, bei dem die Mehrzahl von Halbleiterschaltern (410, 412, 414, 416) eine Mehrzahl von isolierten Gatebipolartransistoren (IGBTs) aufweist.

12. Elektroporationsgenerator nach Anspruch 10, ferner mit einer Mehrzahl von Gatetreibern (438, 440, 442, 444), die zwischen den Mikrocontroller und einen entsprechenden von der Mehrzahl von Halbleiterschaltern (410, 412, 414, 416) geschaltet sind, wobei die Mehrzahl von Gatetreibern (438, 440, 442, 444) konfiguriert ist zum Liefern eines Gateansteuerstroms basierend auf mindestens einem Signal von dem Mikrocontroller (402).

13. Elektroporationsgenerator nach Anspruch 12, ferner mit einer Gatetreiberimpedanz, die zwischen die Mehrzahl von Gatetreibern und einem entsprechenden von der Mehrzahl von Halbleiterschaltern geschaltet ist, wobei die Gatetreiberimpedanz konfiguriert ist zum Kalibrieren einer Anstiegszeit für einen Hochspannungs-DC, der durch die Mehrzahl von Halbleiterschaltern geliefert wird, und ferner konfiguriert ist zum Reduzieren einer Schwingungsantwort durch die Mehrzahl von Halbleiterschaltern.

14. Verfahren zum Erzeugen eines Pulssignals, wobei das Verfahren aufweist:
Liefern einer positiven Hochspannungsgleichstrom (+HVDC) Versorgung (1008) mit einer ersten Polarität und mit einer Spannungsamplitude von mindestens 500 Volt an eine Mehrzahl von Halbleiterschaltern (410, 412, 414, 416), die in einer Brückenkonfiguration verbunden sind;
Liefern einer negativen Hochspannungsgleichstrom (-HVDC) Versorgung (1012) mit einer zweiten Polarität, die der ersten Polarität entgegengesetzt ist, an die Mehrzahl von Halbleitern (410, 412, 414, 416) und mit einer Spannungsamplitude von mindestens 500 Volt;
Kommutieren der Mehrzahl von Halbleiterschaltern (410, 412, 414, 416) zum Anlegen der +HVDC-Versorgung (1012) an einen ersten Leiter (422) eines Katheters (14) und Anlegen der -HVDC-Versorgung (1012) an einen zweiten Leiter (424) des Katheters (14) für eine erste Dauer, die kleiner als 20 Mikrosekunden ist;
Kommutieren der Mehrzahl von Halbleiterschaltern (410, 412, 414, 416) nach der ersten Dauer, zum elektrischen Trennen des ersten Leiters (422) und des zweiten Leiters (424) von der +HVDC-Versorgung (1008) und der -HVDC-Versorgung (1012) für eine zweite Nicht-Null-Zeitdauer;
Kommutieren der Mehrzahl von Halbleiterschaltern (410, 412', 414, 416), nach der zweiten Nicht-Null-Zeitdauer, zum Anlegen der +HVDC-Versorgung (1008) an den zweiten Leiter (424) und zum Anlegen der -HVDC-Versorgung (1012) an den ersten Leiter (422) für eine dritte Dauer, die kleiner als 20 Mikrosekunden ist;
Kommutieren der Mehrzahl von Halbleiterschaltern (410, 412, 414, 416) nach der dritten Dauer zum elektrischen Trennen des ersten Leiters (422) und des zweiten Leiters (424) von der +HVDC-Versorgung (1008) und der -HVDC-Versorgung (1012), wobei das Kommutieren der Mehrzahl von Halbleiterschaltern (410, 412, 414, 416) während der ersten bis dritten Dauer ein Zweiphasenpulssignal (700) durch den ersten und zweiten Leiter (422, 424) überträgt, wobei das Zweiphasenpulssignal eine erste Phase mit der ersten Polarität und der ersten Zeitdauer und eine zweite Phase mit der zweiten Polarität und der zweiten Zeitdauer aufweist; und
Verhindern, dass Rauschen, das durch das Kommutieren der Mehrzahl von Halbleiterschaltern (410, 412, 414, 416) erzeugt wird, den Mikrocontroller (402) erreicht, indem ein Opto-Isolator (1026) verwendet wird, der den Mikrocontroller (402) kommunikativ mit jedem von der Mehrzahl von Halbleiterschaltern (410, 412, 414, 416) koppelt.

## Revendications

1. Système d'électroporation comprenant:
une tige de cathéter (44);
un ensemble d'électrodes (200) couplé à la tige de cathéter à son extrémité distale, l'ensemble d'électrodes comprenant au moins une électrode, dans lequel l'ensemble d'électrodes est configuré comme un élément parmi un ensemble de boucle d'électrode et un ensemble d'électrodes planes; et
un générateur d'électroporation (26) couplé en communication avec ledit au moins une électrode, le générateur d'électroporation (26) configuré pour fournir un signal d'impulsion biphasique (700) à ladite au moins une électrode, le signal d'impulsion biphasique (700) comprenant les éléments suivants:
une première phase (702) ayant une première polarité et une première durée d'impulsion; et
une deuxième phase (708) ayant une deuxième polarité opposée à la première polarité, et une deuxième durée d'impulsion, chacune de la première phase (702) et de la deuxième phase (708) ayant une amplitude de tension d'au moins 500 volts et une durée d'impulsion inférieure à 20 microsecondes,
dans lequel la deuxième phase (708) est générée à un intervalle non nul après la première phase (702), et
dans lequel le générateur d'électroporation (26) comprend:
un microcontrôleur (402);
une pluralité d'éléments de commutation (410, 412, 414, 416); et
un opto-isolateur (1026) couplant de manière communicative le microcontrôleur (402) avec chacun de la pluralité d'éléments de commutation et configuré pour empêcher le bruit généré par la commutation de la pluralité d'éléments de commutation d'atteindre le microcontrôleur.

2. Système d'électroporation de la revendication 1, dans lequel chacune de la première et de la deuxième phase a une amplitude de tension dans la gamme de 500 V à 3,5 kV, 600 V à 2,5 kV, 800 V à 3,5 kV, 600 V à 2,0 kV, 800 V à 2,5 kV, 1,0 kV à 3,5 kV, 600 V à 1,5 kV, 800 V à 2,0 kV, ou 1,0 kV à 2,5 kV
et une durée d'impulsion dans la gamme de 1 nanoseconde à 20 microsecondes, ou
une durée d'impulsion dans la gamme de 0,1 microseconde à 20 microsecondes, ou
une durée d'impulsion dans la gamme de 1 nanoseconde à 10 microsecondes, ou
une durée d'impulsion dans la gamme de 0,1 microseconde à 15 microsecondes, ou
une durée d'impulsion dans la gamme de 1 nanoseconde à 5 microsecondes, ou
une durée d'impulsion dans la gamme de 0,1 microseconde à 10 microsecondes, ou
une durée d'impulsion dans la gamme de 0,1 microseconde à 5 microsecondes, ou
une durée d'impulsion inférieure à 1,5 microseconde, ou
une durée d'impulsion inférieure à 5 microsecondes, ou
une durée d'impulsion inférieure à 4 microsecondes, ou
une durée d'impulsion inférieure à 3 microsecondes, ou
une durée d'impulsion inférieure à 2 microsecondes, ou
une durée d'impulsion inférieure à 1 nanoseconde, ou
dans lequel chacun des éléments suivants: la première durée d'impulsion, la deuxième durée d'impulsion et l'intervalle non nul sont inférieurs à 3 microsecondes, ou dans lequel chacun des éléments suivants: la première durée d'impulsion, la deuxième durée d'impulsion et l'intervalle non nul sont inférieurs à 3 microsecondes et chacun des éléments suivants: la première phase et la deuxième phase ont une amplitude de tension d'au moins 600 volts.

3. Système d'électroporation de la revendication 1 ou 2, dans lequel le générateur d'électroporation (26) est en outre configuré pour générer une pluralité de signaux d'impulsion biphasiques, dans lequel chacun de la pluralité de signaux d'impulsion biphasiques est généré à une période d'impulsion dans la gamme de 0,5 milliseconde à 10 millisecondes.

4. Système d'électroporation de la revendication 1, dans lequel l'ensemble d'électrodes est configuré comme un ensemble de boucles d'électrodes bipolaires, et dans lequel chacune de la première et de la deuxième phase a une amplitude de tension d'au moins 600 volts et une durée d'impulsion inférieure à 3 microsecondes.

5. Système d'électroporation de la revendication 4, dans lequel chacune de la première et la deuxième phase ont une amplitude de tension dans la gamme de 600 volts à 1,4 kV, et une durée d'impulsion dans la gamme de 1 microseconde à 1,5 microseconde.

6. Système d'électroporation de la revendication 1, dans lequel l'ensemble d'électrodes est configuré comme un ensemble de boucles d'électrodes monopolaire, et dans lequel chacune de la première et de la deuxième phase a une amplitude de tension d'au moins 800 volts et une durée d'impulsion inférieure à 3 microsecondes.

7. Système d'électroporation de la revendication 6, dans lequel chacune de la première et de la deuxième phase a une amplitude de tension dans la gamme de 800 volts à 3,0 kV, et une durée d'impulsion dans la gamme de 0,5 microseconde à 1,5 microseconde.

8. Système d'électroporation de la revendication 1, dans lequel l'ensemble d'électrodes (200) est configuré comme un ensemble d'électrodes expansibles bipolaires comprenant un élément d'isolation expansible, et dans lequel chacune de la première et de la deuxième phase a une amplitude de tension d'au moins 500 volts et une durée d'impulsion inférieure à 20 microsecondes.

9. Système d'électroporation de la revendication 8, dans lequel chacune de la première et de la deuxième phase a une amplitude de tension dans la gamme de 600 volts à 2,5 kV, et une durée d'impulsion dans la gamme de 500 nanosecondes à 1,5 microseconde.

10. Générateur d'électroporation (26) comprenant:
une alimentation en courant continu à haute tension positive (+HVDC) (1008) ayant une première polarité;
une alimentation en courant continu à haute tension négative (-HVDC) (1012) ayant une deuxième polarité opposée à la première polarité;
une pluralité de commutateurs semi-conducteurs (410, 412, 414, 416) connectés dans une configuration de pont pour réguler l'application de l'alimentation +HVDC (1008) et de l'alimentation -HVDC (1012) aux premier et deuxième conducteurs (422, 424) pour un cathéter (14); et
un microcontrôleur (402) couplé de manière communicative à la pluralité de commutateurs semi-conducteurs (410, 412, 414, 416) et configuré pour commander la commutation de la pluralité de commutateurs semi-conducteurs (410, 412, 414, 416) afin de transmettre un signal d'impulsion biphasique (500) à travers les premier et deuxième conducteurs (422, 424) pour le cathéter (14), dans lequel le microcontrôleur (402) est en outre configuré pour générer le signal d'impulsion biphasique (500) comprenant:
une première phase ayant la première polarité et une première durée d'impulsion;
une deuxième phase ayant la deuxième polarité et une deuxième durée d'impulsion, chacune de la première et de la deuxième phase ayant une amplitude de tension d'au moins 500 volts et une durée d'impulsion inférieure à 20 microsecondes; et
dans lequel la deuxième phase est générée à un intervalle non nul après la première phase; et
un opto-isolateur (1026) couplant de manière communicative le microcontrôleur (402) couplant de manière communicative le microcontrôleur (402) avec chacun de la pluralité de commutateurs semi-conducteurs et configuré pour empêcher le bruit généré par la commutation de la pluralité de commutateurs semi-conducteurs d'atteindre le microcontrôleur.

11. Générateur d'électroporation (26) selon la revendication 10, dans lequel la pluralité de commutateurs semi-conducteurs (410, 412, 414, 416) comprend une pluralité de transistors bipolaires à grille isolée.

12. Générateur d'électroporation de la revendication 10 comprend en outre une pluralité de commande de grille (438, 440, 442, 444) couplées entre le microcontrôleur et les commutateurs semi-conducteurs correspondants de la pluralité de commutateurs semi-conducteurs (410, 412, 414, 416), dans lequel la pluralité de commande de grille (438, 440, 442, 444) est configurée pour fournir un courant de commande de grille sur la base d'au moins un signal provenant du microcontrôleur (402).

13. Générateur d'électroporation de la revendication 12 comprend en outre une impédance de commande de grille couplée entre chacun de la pluralité de commandes de grille et les commutateurs correspondants de la pluralité de commutateurs semi-conducteurs, l'impédance de commande de grille étant configurée pour calibrer un temps de montée pour le courant continu à haute tension fourni à travers la pluralité de commutateurs à semi-conducteurs, et étant en outre configurée pour réduire la réponse oscillatoire de la pluralité de commutateurs semi-conducteurs.

14. Procédé de génération d'un signal d'impulsion, comprenant les étapes consistant à:
fournir une alimentation en courant continu à haute tension positive (+HVDC) (1008) ayant une première polarité et une amplitude de tension d'au moins 500 volts à une pluralité de commutateurs semi-conducteurs (410, 412, 414, 416) connectés dans une configuration de pont;
fournir une alimentation en courant continu à haute tension négative (-HVDC) (1012) ayant une deuxième polarité opposée à la première polarité à la pluralité de semi-conducteurs (410, 412, 414, 416) et ayant une amplitude de tension d'au moins 500 volts;
commuter la pluralité de commutateurs semi-conducteurs (410, 412, 414, 416) pour appliquer l'alimentation +HVDC (1012) à un premier conducteur (422) d'un cathéter (14) et pour appliquer l'alimentation -HVDC (1012) à un deuxième conducteur (424) du cathéter (14) pendant une première durée inférieure à 20 microsecondes;
commuter la pluralité de commutateurs semi-conducteurs (410, 412, 414, 416), après la première durée, pour déconnecter électriquement le premier conducteur (422) et le deuxième conducteur (424) de l'alimentation +HVDC (1008) et de l'alimentation - HVDC (1012) pendant une seconde durée non nulle;
commuter la pluralité de commutateurs semi-conducteurs (410, 412, 414, 416), après la deuxième durée non nulle, pour appliquer l'alimentation +HVDC (1008) au deuxième conducteur (424) et appliquer l'alimentation -HVDC (1012) au premier conducteur (422) pendant une troisième durée inférieure à 20 microsecondes;
commuter la pluralité de commutateurs semi-conducteurs (410, 412, 414, 416), après la troisième durée, pour déconnecter électriquement le premier conducteur (422) et le deuxième conducteur (424) de l'alimentation +HVDC (1008) et de l'alimentation - HVDC (1012), dans lequel la commutation de la pluralité de commutateurs semi-conducteurs (410, 412, 414, 416) pendant la première à la troisième durée transmet un signal d'impulsion biphasique (700) à travers les premier et deuxième conducteurs (422, 424), le signal d'impulsion biphasique comprenant une première phase ayant la première polarité et la première durée et une deuxième phase ayant la deuxième polarité et la deuxième durée; et
empêcher le bruit généré par la commutation de la pluralité de commutateurs semi-conducteurs (410, 412, 414, 416) d'atteindre le microcontrôleur (402) à l'aide d'un opto-isolateur (1026) qui couple de manière communicative le microcontrôleur (402) avec chacun de la pluralité de commutateurs semi-conducteur, (410, 412, 414, 416).
